# EUROPEAN PATENT APPLICATION

(11) **EP 0 712 859 A1**
(43) Date of publication of application: **22.05.1996**
(21) Application number: 94500173.3
(22) Date of filing: 08.11.1994
(51) Int. Cl.: C07H 15/24, C12P 19/44, C12R 1/645, A61K 31/705

(54) **Antifungal Sordarin derivatives**

(71) Applicant: GLAXO, S.A., E-28760 Tres Cantos, Madrid (ES)
(72) Inventor: Dawson, Michael, Greenford, Middlesex UB6 0HE (GB); Hall, Richard, Greenford, Middlesex UB6 0HE (GB); Castro Pichel, Julia, E-28760 Tres Cantos, Madrid (ES)
(74) Representative: Carpintero Lopez, Francisco

(57) **Abstract**

Sordarin derivatives of formula (I) 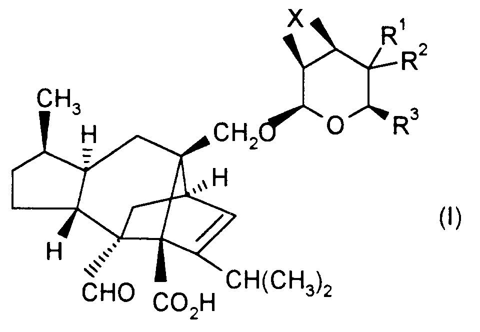
have antifungal activity.

## Description

This invention relates to novel sordarin derivatives having antifungal activity, to processes for their preparation, to pharmaceutical compositions containing them and to their use in medicine, more particularly in the prevention or treatment of diseases in animals, including humans, caused by fungal infection.

British Patent Specification No. 1,162,027 describes the preparation of an antibiotic, SL2266, by the cultivation of the strain NRRL 3196 of the fungus species Sordaria araneosa. SL 2266, later named sordarin, is reported to have fungistatic activity. The same research group also described in Helvetica Chimica Acta (1971), 51, 119-120 the degradation of sordarin to sordaricin. Published Japanese Patent Application No. J6 2040292A describes the preparation of an antibiotic, zofimarin, which is reported to have antifungal activity.

Sordarin, sordaricin and zofimarin may be represented by formula (A) below 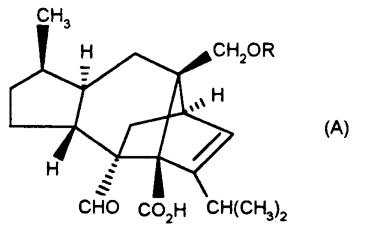
where OR as 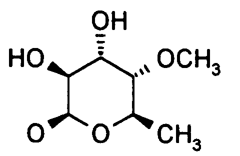
describes sordarin; OR as OH describes sordaricin; and OR as 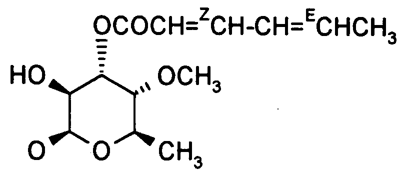
describes zofimarin.

Although sordarin and zofimarin exhibit antifungal activity, both compounds are only moderately active and have limited spectra of action when tested against a battery of fungal organisms. We now describe hereinafter a novel group of fungicidal sordarin derivatives which exhibit excellent antifungal activity and a broad spectrum of action. Thus, according to a first aspect of the present invention, we provide compounds of formula (I) 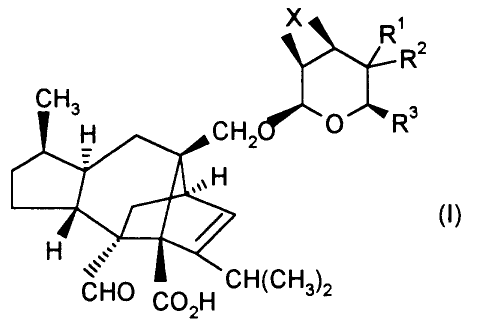
and pharmaceutically acceptable salts and solvates (e.g. hydrates) thereof, wherein R¹ represents hydrogen, halogen, C₁₋₆alkyl, hydroxy, C₁₋₆alkoxy, C₁₋₄alkoxyC₁₋₄alkoxy, arylC₁₋₄alkoxy or a group OCOR⁴ (where R⁴ is arylC₁₋₄alkoxy or a C₁₋₁₀alkyl group optionally containing one or two double bonds) and R² represents hydrogen or R¹ and R² may together with the carbon atom to which they are attached represent C=O;

R³ represents CH₂R⁵ (where R⁵ is hydrogen, hydroxyl, C₁₋₄alkoxy or a group OCOR⁶ in which R⁶ is C₁₋₄alkyl); and

X represents an oxygen or sulphur atom or a CH₂ group.

Suitable pharmaceutically acceptable salts of the compounds of formula (I) include inorganic base salts such as alkali metal salts (for example sodium and potassium salts) and ammonium salts and organic base salts. Suitable organic base salts include amine salts such as trialkylamine (e.g. triethylamine), dialkylamine (e.g. dicyclonexylamine), optionally substituted benzylamine (e.g. phenylbenzylamine or p-bromobenzylamine), procaine, ethanolamine, diethanolamine, N-methylglucosamine and tri(hydroxymethyl)methylamine salts and amino acid salts (e.g. lysine and arginine salts).

References hereinafter to a compound of formula (I) includes that compound and its pharmaceutically acceptable salts.

Other salts which are not pharmaceutically acceptable may be useful in the preparation of compounds of formula (I) and these form a further aspect of the invention.

It is to be understood that the present invention encompasses any individual isomers, including optical isomers, of compounds represented by formula (I) above as well as mixtures thereof, including wholly or partially racemic mixtures thereof.

As used herein, the term "alkyl" as a group or part of a group means a straight or branched chain alkyl moiety comprising, for example, 1 to 8 carbon atoms. Suitable examples include methyl, ethyl, n-propyl, i-propyl, n-butyl, s-butyl, t-butyl, n-hexyl and n-octyl.

As used herein, the term "aryl" as a group or part of a group means phenyl or heteroaryl each optionally substituted by one or more (e.g. 1, 2 or 3) atoms or groups selected from halogen, hydroxyl, C₁₋₆alkyl, C₁₋₆alkoxy or C₁₋₄alkoxycarbonyl. The heteroaryl group may be a 5- or 6-membered heteroaromatic ring containing one or more heteroatoms selected from nitrogen, oxygen and sulphur. Suitable examples of heteroaryl groups include pyridyl, furyl, thienyl and pyrrolyl.

The term "halogen" means herein fluorine, chlorine, bromine or iodine.

When R⁴ is an unsaturated C₁₋₁₀alkyl group it may particularly represent a straight or branched chain C₅₋₈alkyl group containing two double bonds, for example -CH=^{Z}CH-CH=^{E}CHCH₃

R² is preferably a hydrogen atom with R¹ sited in the α-configuration.

Examples of particular R¹ groups include halogen (e.g. fluorine), hydroxyl, C₁₋₄alkoxy, benzyloxy, benzyloxycarbonyloxy, C₁₋₄alkoxycarbonyloxy and C₁₋₄alkoxyC₁₋₄alkoxy (e.g. methoxyethoxy).

R³ may represent, for example, methyl or C₁₋₄alkoxymethyl (e.g. methoxymethyl).

R¹ preferably represents C₁₋₄alkoxy, benzyloxy or OCOR⁴ (where R⁴ is a C₁₋₄alkyl group), especially sited in the α-configuration.

R³ preferably represents methyl.

X preferably represents oxygen.

It is to be understood that the present invention covers all combinations of particular and preferred groups described hereinabove.

A particular group of compounds of the invention are compounds of formula (Ia) 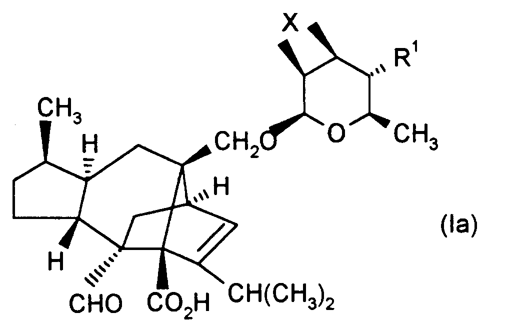
and pharmaceutically acceptable salts and solvates (e.g. hydrates) thereof, wherein X and R¹ are as defined previously, especially where X is oxygen and R¹ is a group selected from C₁₋₄alkoxy, benzyloxy or OCOR⁴ (where R⁴ is a C₁₋₄alkyl group).

Specific compounds according the present invention include:
[1R-(1α, 3aβ, 4β, 4aβ, 7β, 7aα, 8aβ)] 8a-[(2,3-anhydro-6-deoxy-4-O-propyl-β-D-mannopyranosyloxy)methyl]-4-formyl-4,4a,5,6,7,7a,8,8a-octahydro-7-methyl-3-(1-methylethyl)-1,4-methano-*s*-indacene-3a(1H)-carboxylic acid; [1R-(1α, 3aβ, 4β, 4aβ, 7β, 7aα, 8aβ)] 8a-[(2,3-anhydro-6-deoxy-4-O-methyl-β-D-mannopyranosyloxymethyl]-4-formyl-4,4a,5,6,7,7a,8,8a-octahydro-7-methyl-3-(1-methylethyl)-1,4-methano-*s*-indacene-3a(1H)-carboxylic acid; and pharmaceutically acceptable salts and solvates (e.g. hydrates) thereof.

The compounds of formula (I) are very active fungicides useful in combatting fungal infections in animals, including humans. For example, they may be used in the treatment of systemic infections caused by organisms such as species of Candida (e.g. Candida albicans, Candida glabrata, (Torulopsis glabrata), Candida tropicalis, Candida paransilosis and Candida pseudotropicalis), Cryptococcus neoformans, Pneumocystis carinii, Aspergillus Sp (e.g. Aspergillus flavus and Aspergillus fumigatus), Coccidioides (e.g. Coccidioides immitis), Paracoccidioides (e.g. Paracoccidioides brasiliensis), Histoplasma (e.g. Histoplasma capsulatum) or Blastomyces (e.g. Blastomyces dermatitidis). They may also be used to treat topical infections caused by species of Candida, Trichophyton, Microsporum or Epidermophyton (e.g. Trichophyton mentographytes, Trichophyton rubrum, Microsporum canis or Epidermophyton floccosum), or in mucosal infections caused by Candida albicans.

Compounds of formula (I) may also be used to treat other infections caused by species of filamentous fungi such as Geotrichum (e.g. Geotrichum clavatum), Trichosporon (e.g. Trichosporon beigelii), Blastoschizomyces (e.g. Blastoschizomyces capitatus), Sporothrix (e.g. Sporothrix schenckii), Scedosporium (e.g. Scedosporium apiosperum), Cladosporium (e.g. Cladosporium carrionii) and Pityrosporum ovale.

The in vitro evaluation of the anti-fungal activity of compounds of the invention was performed on liquid or solid medium by the anti-fungal two-fold serial dilution technique of determining the minimum inhibitory concentration (MIC) of anti-fungal agent that inhibited development of growth after 24 to 48 hours of incubation at 37°C. In practice, a series of agar plates or broth microdilution panels containing two-fold dilutions of anti-fungal agent tested were inoculated with a standard culture of a clinically relevant pathogen, for example, candida albicans. The agar plates or broth microdulution panels were then examined for the presence or absence of growth of the fungus and the appropriate MIC values were noted.

MFC values (defined as the lowest anti-fungal concentration that killed at least 99.9% of the initial inoculum in liquid medium) may also be determined by sub-culturing 0.01 and 0.1 µl of broth from the drug-free control well, the first well containing growth and each clear well on agar plates.

The in vivo evaluation of compounds of formula (I) can be carried out at a series of dose levels by administration (e.g. subcutaneously, orally, intraperitoneally or intravenously) to mice inoculated with a strain of Candida albicans. Untreated mice die within 3 to 9 days and the dose level at which the test compound provides 50% protection against the lethal effect of the infection is noted.

In view of their antifungal activity, compounds of formula (I) recommend themselves for the treatment of a variety of fungal infections in human beings and animals. Such infections include superficial, cutaneous, subcutaneous and systemic mycotic infections such as respiratory tract infections, gastrointestinal tract infections, cardiovascular infections, urinary tract infections, CNS infections, candidiasis and chronic mucocandidiasis (e.g. thrush and vaginal candidiasis) and skin infections caused by fungi, cutaneous and mucocutaneous candidiasis, dermatophytoses including ringworm and tinea infections, athletes foot, paronychia, pityriasis versicolor, erythrasma, intertrigo, fungal nappy rash, candida vulvitis, candida balanitis and otitis externa. They may also be used as prophylactic agents to prevent systemic and topical fungal infections. Use as prophylactic agents may, for example, be appropriate as part of a selective gut decontamination regimen in the prevention of infection in immunocompromised patients (e.g. AIDS patients, patients receiving cancer therapy or transplant patients). Prevention of fungal overgrowth during antibiotic treatment may also be desirable in some disease syndromes or iatrogenic states.

While it is possible that, for use in therapy, compounds of the invention may be administered as the raw chemical, it is preferable to present the active ingredient as a pharmaceutical formulation. The invention thus further provides a pharmaceutical formulation comprising compounds of formula (I) and physiologically acceptable salts thereof together with one or more pharmaceutically acceptable carriers thereof and, optionally, other therapeutic and/or prophylactic ingredients. The carrier(s) must be 'acceptable' in the sense of being compatible with the other ingredients of the formulation and not deleterious to the recipient thereof.

The compositions of the invention include those in a form especially formulated for oral, buccal, parenteral, implant, rectal, topical, ophthalmic or genito-urinary administration or in a form suitable for administration by inhalation or insufflation.

Tablets and capsules for oral administration may contain conventional excipients such as binding agents, for example, syrup, acacia, gelatin, sorbitol, tragacanth, mucilage of starch or polyvinylpyrrolidone; fillers, for example, lactose, sugar, microcrystalline cellulose, maize-starch, calcium phosphate or sorbitol; lubricants, for example, magnesium stearate, stearic acid, talc, polyethylene glycol or silica; disintegrants, for example, potato starch or sodium starch glycollate; or wetting agents such as sodium lauryl sulphate. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, for example, sorbitol syrup, methyl cellulose, glucose/sugar syrup, gelatin, hydroxyethylcellulose, carboxymethyl cellulose, aluminium stearate gel or hydrogenated edible fats; emulsifying agents, for example, lecithin, sorbitan mono-oleate or acacia; non-aqueous vehicles (which may include edible oils), for example, almond oil, fractionated coconut oil, oily esters, propylene glycol or ethyl alcohol; and preservatives, for example, methyl or propyl p-hydroxybenzoates or sorbic acid. The compositions may also be formulated as suppositories, e.g. containing conventional suppository bases such as cocoa butter or other glycerides.

For buccal administration the composition may take the form of tablets or lozenges formulated in conventional manner.

The composition according to the invention may be formulated for parenteral administration by injection or continuous infusion. Formulations for injection may be presented in unit dose form in ampoules, or in multi-dose containers with an added preservative. The compositions may take such forms as suspensions, solutions, or emulsions in oily or aqueous vehicles, and may contain formulatory agents such as suspending, stabilising and/or dispersing agents. Alternatively the active ingredient may be in powder form for constitution with a suitable vehicle, e.g. sterile, pyrogen-free water, before use.

For administration by inhalation the compositions according to the invention are conveniently delivered in the form of an aerosol spray presentation from pressurised packs with the use of a suitable propellant, e.g. dichlorodifluoromethane, trichlorofluoromethane, dichlorotetrafluoroethane, carbon dioxide or other suitable gas, or from a nebuliser. In the case of a pressurised aerosol the dosage unit may be determined by providing a valve to deliver a metered amount.

Alternatively, for administration by inhalation the compositions according to the invention may take the form of a dry powder composition, for example a powder mix of the compound and a suitable powder base such as lactose or starch. The powder composition may be presented in unit dosage form in, for example, capsules or cartridges of e.g. gelatin, or blister packs from which the powder may be administered with the aid of an inhaler or insufflator.

The compositions may take the form of a suppository, e.g. containing a conventional suppository base, or a pessary, e.g. containing a conventional pessary base.

The compositions may also be formulated for topical administration in the form of ointments, creams, gels, lotions, shampoos, powders (including spray powders), pessaries, tampons, sprays, dips, aerosols, drops (e.g. eye, ear or nose drops) or pour-ons. Ointments and creams may, for example, be formulated with an aqueous or oily base with the addition of suitable thickening and/or gelling agents. Ointments for administration to the eye may be manufactured in a sterile manner using sterilised components. Pour-ons may, for example, be formulated for veterinary use in oils containing organic solvents, optionally with formulatory agents, e.g. stabilising and solubilising agents. Pessaries and tampons for vaginal insertion may be formulated using conventional techniques and, where appropriate, may contain an effervescent vehicle. Such compositions may also contain other active ingredients such as corticosteroids, antibiotics or antiparasitics as appropriate.

Liquid preparations for intranasal delivery may take the form of solutions or suspensions and may contain conventional excipients such as tonicity adjusting agents, for example, sodium chloride, dextrose or mannitol; preservatives, for example benzalkonium chloride, thiomersal, phenylethyl alcohol; and other formulating agents such as suspending, buffering, stabilising and/or dispersing agents.

Transdermal administration may be affected by the design of a suitable system which promotes adsorption of the active compound through the skin and would typically consist of a base formulation enclosed within an adhesive stick-on patch comprising backing films, membranes and release liners.

The composition according to the invention may also be formulated as a depot preparation. Such long acting formulations may be administered by implantation (for example subcutaneously or intramuscularly) or by intramuscular injection. Thus, for example, a compound of the invention may be formulated with suitable polymeric or hydrophobic materials (for example as an emulsion in an acceptable oil) or ion exchange resins, or as sparingly soluble derivatives, for example, as a sparingly soluble salt.

When the compositions comprise dosage units, each unit will preferably contain 0.001mg to 1000mg, advantageously 0.01mg to 400mg, of active ingredient where a compound of the invention is to be administered orally. The daily dosage as employed for adult human treatment will preferably range from 0.001mg to 5000mg of active ingredient, most preferably from 0.01mg to 2000mg which may be administered in 1 to 4 daily doses, for example, depending on the route of administration and on the condition of the patient and the disease to be treated.

The compound may be administered by intravenous infusion using, for example, up to 50mg/kg/day of the active ingredient. The duration of treatment will be dictated by the rate of response rather than by arbitrary numbers of days.

Compounds of the invention may also be used in combination with other therapeutic agents, and the invention thus provides, in a further aspect, a combination comprising a compound of the invention together with another therapeutically active agent.

The combinations referred to above may conveniently be presented for use in the form of a pharmaceutical formulation and thus pharmaceutical formulations comprising a combination as defined above together with a pharmaceutically acceptable carrier thereof comprise a further aspect of the invention. The individual components of such combinations may be administered either sequentially or simultaneously in separate or combined pharmaceutical formulations.

When a compound of the invention is used in combination with a second therapeutic agent against the same condition the dose of each compound may differ from that when the compound is used alone. Appropriate doses will be readily appreciated by those skilled in the art.

According to another aspect of the present invention, we provide a compound of formula (I) or a physiologically acceptable salt thereof or a pharmaceutical composition comprising a compound of formula (I) or a physiologically acceptable salt thereof as defined above for use in therapy, particularly for the treatment of fungal infections in animals (especially humans).

According to another aspect of the present invention, we provide the use of a compound of formula (I) or a physiologically acceptable salt thereof in the manufacture of a medicament for the treatment of fungal infections in a human or non-human animal patient.

According to a further aspect of the present invention, we provide a method of treatment of the human or non-human animal body to combat fungal diseases, which method comprises administering to said body an effective amount of a compound of formula (I) or a physiologically acceptable salt thereof.

It will be appreciated by those skilled in the art that references herein to treatment extend to prophylaxis as well as the treatment of established conditions or infections.

The compounds of the invention may be prepared by the processes described below.

Thus, a general process (A) for the preparation of a compound of formula (I) in which X is oxygen comprises cyclising a compound of formula (II) 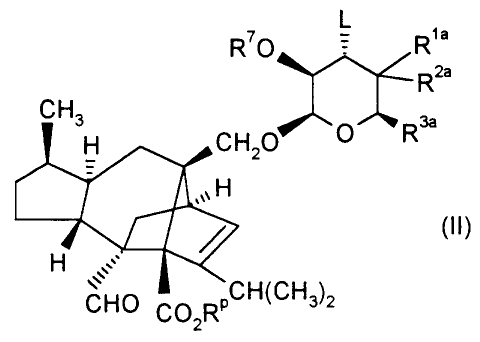
(in which R^{1a}, R^{2a} and R^{3a} are as defined for R¹, R² and R³ in formula (I) above or are protected derivatives thereof, R^{p} is hydrogen or a carboxyl protecting group, L is a suitable leaving group such as an alkyl- or arylsulphonyloxy group and R⁷ is hydrogen or OR⁷ is the same group as defined for L), followed, where necessary, by the removal of any protecting groups present.

The cyclisation reaction may conveniently be effected by treating a compound of formula (II) with a strong base such as an alkali metal hydride (e.g. sodium hydride) in a suitable solvent such as dimethylformamide or an ether (e.g. tetrahydrofuran), conveniently at about room temperature. Alternatively, sodium in an alcoholic solvent (e.g. methanol) may be used, especially when OR⁷ is the same group as defined for L. In this instance the base system is added to a solution of a compound of formula (II) in a suitable solvent such as a halogenated hydrocarbon (e.g. dichloromethane) and the reaction is conveniently carried out at a temperature of from room temperature to reflux.

Another general process (B) for the preparation of a compound of formula (I) in which X is sulphur comprises treating a compound of formula (III) 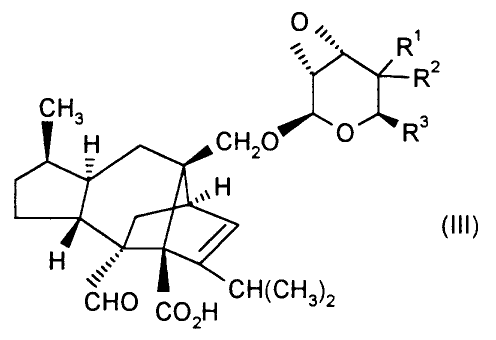
(in which R¹, R² and R³ are as defined in formula (I) above) with a sulphur donor. Thus, for example, the reaction may conveniently be effected by treating a compound of formula (III) with 5,5-dimethyl-2-thiolo-2-thioxo-1,3,2-dioxaphosphorinane in a solvent such as dimethylformamide, preferably in the presence of a suitable base such as a trialkylamine (e.g. triethylamine) at an elevated temperature (e.g. about 80° to 120°C).

A compound of formula (III) may conveniently be prepared from a compound of formula (II) in which L is a hydroxyl group and OR⁷ represent a suitable leaving group such as an alkyl- or arylsulphonyloxy group using the conditions described in process (A), followed, where necessary, by the removal of any protecting groups present.

Another general process (C) comprises an interconversion reaction wherein a compound of formula (I) is prepared from a different compound of formula (I) or a protected derivative thereof.

According to a first embodiment of process (C), a carboxyl protected derivative of a compound of formula (I) in which R¹ is hydroxyl may be converted to a corresponding compound of formula (I) in which R¹ is halogen by a standard displacement reaction, and thereafter removing the carboxyl protecting group. Thus, for example, the displacement of hydroxyl by a fluorine atom may conveniently be effected with inversion of configuration by adding diethylaminosulphur trifluoride (DAST) to a solution of the starting material in a solvent such as a halogenated hydrocarbon (e.g. dichloromethane). The reaction may conveniently be effected at about room temperature.

In a further embodiment of process (C), a compound of formula (I) in which R¹ is C₁₋₆alkoxy or C₁₋₄alkoxyC₁₋₄alkoxy and/or R³ is C₁₋₄alkoxymethyl may be prepared by alkylating a protected derivative of a compound of formula (I) in which R¹ and/or R³ contains a free hydroxyl group and any labile groups (e.g. carboxyl and hydroxyl groups) are protected as appropriate, followed by removal of the protecting groups present. The alkylation may conveniently be effected by initial reaction with a strong alkali metal base (e.g. sodium hydride) and thereafter with an alkyl halide (e.g. methyl bromide). The reaction may be carried out in a suitable solvent such as an ether (e.g. tetrahydrofuran) at a temperature within the range of about 0° to 50°C. Where appropriate, an ammonium salt such as a tetraalkylammonium halide (e.g. tetrabutylammonium iodide) may also be present.

Alternatively, a straight or branched alkyl group may conveniently be introduced in two steps, the first step comprising alkenylation using a suitable alkenylhalide in the presence of a base such as a carbonate (e.g. cesium carbonate) in a solvent such as dimethylformamide at about room temperature, and the second step comprising a hydrogenation procedure in the presence of a palladium catalyst (e.g. 10% palladium on charcoal) at about room temperature.

In another embodiment of process (C), a compound of formula (I) in which R³ is CH₂OCOR⁶ or R¹ is OCOR⁴ may be prepared by acylating a protected derivative of a compound of formula (I) in which R³ is CH₂OH or R1 is hydroxyl and any labile groups (e.g. carboxyl and hydroxyl groups) are protected, followed by removal of any protecting groups present. The acylation reaction may be carried out using conventional methodology, for example by treatment with a carboxylic acid in the presence of an activating agent such as dicyclohexylcarbodiimide and a suitable base such as dimethylaminopyridine, or using an acid halide (e.g. an acid chloride), optionally in the presence of a suitable base such as pyridine or dimethylaminopyridine.

According to a further embodiment of process (C), a compound of formula (I) in which CR¹R² represents C=O may be prepared by oxidising a protected derivative of a compound of formula (I) in which R¹ is a hydroxyl group and any labile groups (e.g. carboxyl and hydroxyl groups) are protected, followed by removal of any protecting groups present. The oxidation reaction may conveniently be effected by addition of a suitable oxidising agent such as dimethylsulphoxide in the presence of trifluoroacetic anhydride. The oxidation conveniently takes place in the presence of a suitable solvent such as a halogenated hydrocarbon (e.g. dichloromethane) at an elevated temperature (e.g. about 40° to 80°C).

Another general process (D) for the preparation of a compound of formula (I) comprises treating a compound of formula (IV) 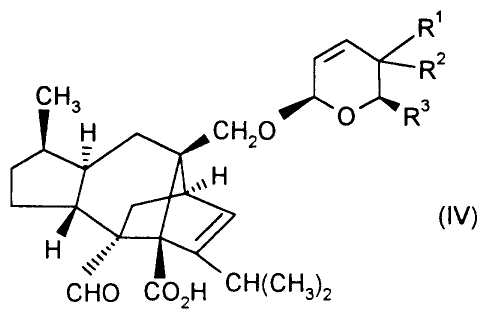
or a protected derivative thereof with a reagent capable of donating an oxygen atom or a CH₂ group, followed by the removal of any protecting groups present.

In one embodiment of process (D), a compound of formula (I) in which X is oxygen may be prepared by treating a compound of formula (IV) with a peroxide oxidising agent such as a hydroperoxide acid (e.g. m-chloroperbenzoic acid) in a suitable solvent such as a hydrocarbon (e.g. toluene).

In another embodiment of process (D), a compound of formula (I) in which X is CH₂ may be prepared by treating a compound of formula (IV) with methylene iodide and iodine in the presence of a zinc-copper couple.

A compound of formula (IV) may conveniently be prepared by treating a compound of formula (II) in which L is a hydroxyl group and R⁷ is hydrogen with iodine, the reaction effected at an elevated temperature (e.g. under reflux) in a suitable solvent such as a hydrocarbon (e.g. toluene) and in the presence of triphenylphosphine, imidazole and zinc.

Many of the above-mentioned procedures require the removal of one or more protecting groups as a final step to provide the desired compound of formula (I). Thus, a further general process (E) comprises deprotecting a protected derivative of a compound of formula (I). Suitable carboxyl protecting groups and hydroxyl protecting groups for use herein include any conventional protecting group, for example as described in "Protective Groups in Organic Chemistry", Ed. J. F. W. McOmie (Plenum Press, 1973) or "Protective Groups in Organic Synthesis" by Theodora W. Greene (John Wiley and Sons, 1991). Examples of suitable carboxyl protecting groups include arylalkyl groups such as diphenylmethyl and silyl groups (e.g. trimethylsilylethyl or t-butyldimethylsilyl). Examples of suitable hydroxyl protecting groups include arylalkyl groups such as p-methoxybenzyl and ester groups such as benzyloxycarbonyl. Aldehyde groups may conveniently be protected in the form of cyclic ketals.

The protecting groups may be removed using conventional techniques. Thus, a diphenylmethyl group may conveniently be removed using trifluoroacetic acid or by hydrogenolysis in the presence of a palladium catalyst (e.g. 10% palladium on charcoal). A benzyloxycarbonyl group may conveniently be removed by hydrogenolysis in the presence of a palladium catalyst (e.g. 10% palladium on charcoal). A p-methoxybenzyl group may conveniently be removed using 2,3-dichloro-5,6-dicyano-1,4-benzoquinone. Silyl groups such as trimethylsilylethyl or t-butyldimethylsilyl may conveniently be removed using fluoride ions. Cyclic ketal groups may conveniently be converted to aldehyde group by the addition of a suitable acid such as hydrochloric acid.

Compounds of formula (II) in which R^{3a} is methyl may conveniently be prepared from sordarin or 4'-demethylsordarin, a compound of formula (V) 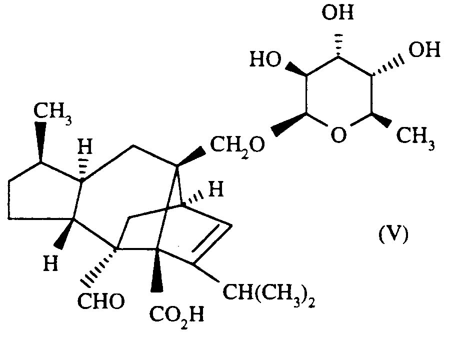
Thus, for example, compounds of formula (II) in which R^{3a} represents methyl may be prepared by protection of the carboxyl group in sordarin or a compound of formula (V) using conventional methods, followed by converting one or more of the hydroxyl groups to the groups specified in formula (II) above. It may be necessary to protect any of the hydroxyl groups when manipulating one or both of the other hydroxyl groups. Suitable protection groups include those hydroxyl protecting groups referred to in process (E) above.

A compound of formula (II) in which R^{3a} is hydroxymethyl may be similarly prepared from 6'-hydroxysordarin, a compound of formula (VI) 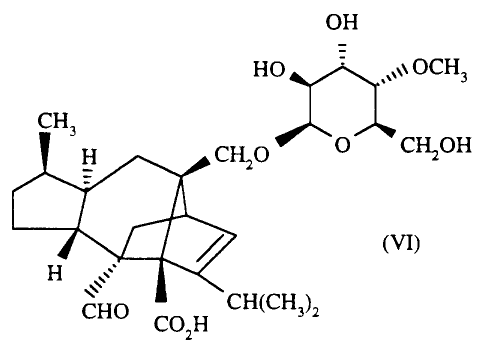
When the carboxyl group in sordarin or a compound of formula (V) or (VI) is protected with a diphenylmethyl group the protection reaction may conveniently be carried out by treating a solution of sordarin or a compound of formula (V) or (VI) in an alcoholic solvent (e.g. methanol) and/or in a halogenated hydrocarbon (e.g. dichloromethane) with diphenyldiazomethane, conveniently added as a solution in a halogenated hydrocarbon solvent (e.g. dichloromethane).

When a hydroxyl group in a carboxyl protected derivative of sordarin or a compound of formula (V) or (VI) is protected with a p-methoxybenzyl group this group may be introduced by reaction with a tin oxide (e.g. dibutyltin oxide) in a hydrocarbon solvent (e.g. refluxing toluene), followed by the addition of a p-methoxybenzyl halide in the presence of an fluoride salt (e.g. tetrabutylammonium fluoride). When protected with a benzyloxycarbonyl group, this group may be introduced by reaction with a benzylhaloformate in the presence of a suitable amine base such as 4-dimethylaminopyridine and in a solvent such as a halogenated hydrocarbon (e.g. dichloromethane) or acetonitrile.

The formation of a group L in formula (II) where L is an alkyl- or arylsulphonyloxy group and/or a group R⁷O where R⁷O is an alkyl- or arylsulphonyloxy group may be effected by reacting a suitably protected derivative of sordarin or a compound of formula (V) or (Vi) with an alkyl- or arylsulphonyl halide in the presence of a suitable solvent such as pyridine and optionally also comprising an amine base (e.g. 4-dimethylaminopyridine) or in a halogenated hydrocarbon solvent (e.g. dichloromethane) in the presence of a suitable amine base (e.g. 4-dimethylaminopyridine). The reaction may conveniently be carried out at about room temperature. Other groups may be similarly introduced using conventional procedures.

It will be appreciated that it may be appropriate to convert a hydroxyl group to the desired R¹ group prior to cyclisation according to process (A). Thus, a suitably protected derivative of 4'-demethylsordarin or a compound of formula (II) in which R^{1a} is hydroxyl may be reacted to convert the 4'-hydroxyl group to the desired R¹ group using conventional procedures. Thus, for example, conversion to a C₁₋₆alkoxy, C₁₋₄alkoxyC₁₋₄alkoxy or arylC₁₋₄alkoxy group may be effected by conventional alkylation, for example, according to the various methods described hereinabove. Removal of the hydroxyl group to provide a compound in which R¹ is hydrogen may conveniently be effected in two steps comprising (i) forming an S-alkyldithiocarbonate by treatment with a strong alkali metal base (e.g. sodium hydride), in the presence of imidazole, and in a suitable solvent such as an ether (e.g. tetrahydrofuran) at a reduced temperature (e.g. about 0°C), and then adding carbon disulphide and an alkylhalide (e.g. methyliodide) at about room temperature and (ii) removing this group by treating a solution of the intermediate compound in a hydrocarbon solvent (e.g. toluene) at an elevated temperature (e.g. about 80° to 120°C) with a hydride reducing agent (e.g. a trialkyltin hydride such as tributyltin hydride), optionally in the presence of an activating agent [e.g. azobis(isobutyronitrile)]. Conversion to a C₂₋₆alkyl group may conveniently be effected by (i) forming an S-alkyldithiocarbonate as described previously (ii) replacing this group with an alkenyl group by reaction with a trialkylalkenyltin compound under the conditions described above for the removal of the S-alkyldithiocarbonate group and (iii) reducing the alkenyl group to an alkyl group by, for example, hydrogenation in the presence of a suitable palladium catalyst (e.g. 10% palladium on charcoal).

Compounds of formulae (II) to (VI) are novel intermediates and form further individual aspects of the present invention. The compound of formula (V), 4'-demethylsordarin, represents a particular aspect of the present invention.

Base salts of compounds of formula (I) may be conveniently formed by treating a compound of formula (I) with an appropriate salt or base. Thus, for example, salts may conveniently be prepared by treating a compound of formula (I) with a salt or a base selected from sodium or potassium hydroxide, hydrogen carbonate, carbonate or acetate (e.g. potassium hydroxide, potassium hydrogen carbonate, sodium hydrogen carbonate or potassium acetate), ammonium acetate, calcium acetate and L-lysine as appropriate. The salt may, for example, be prepared by adding the appropriate salt or base (if necessary as an aqueous solution) to a solution or suspension of the compound of formula (I) in a suitable solvent such as an alcohol (e.g. methanol) or dioxane at temperatures of for example 0°C to 80°C and conveniently at about room temperature.

Pharmaceutically acceptable salts may also be prepared from other salts, including other pharmaceutically acceptable salts of the compounds of formula (I), using conventional methods.

The novel compound of formula (V) may conveniently be prepared according to the fermentation process described hereinafter or by demethylating sordarin using a biotransformation procedure. It is to be understood that such processes for the preparation of the compound of formula (V) represent further aspects of the present invention.

The fermentation process comprises cultivating a microorganism capable of producing the compound of formula (V) and thereafter isolating the compound of formula (V) from the culture.

Microorganisms capable of producing the compound of formula (V) will conveniently be mutant strains of Sordaria araneosa which can be identified by screening survivors of mutagenesis by analysing a test sample obtained from fermentation of the microorganism using standard methodology. In particular, the microorganism to be conveniently used is a mutant strain of Sordaria araneosa deposited in the permanent culture collection of the CAB International Mycological Institute, Genetic Resource Reference Collection, Bakeham Lane, Egham, Surrey TW20 9TY, England. The strain was received by the Institute on 10 June, 1994 and was subsequently given the accession number IMI 362184 and dates of acceptance and confirmation of viability of 13 and 21 June, 1994 respectively. The Institute is an International Depository authority recognised under the Budapest Treaty. The characteristics thus far identified for IMI 362184 are given in Example 15.

The present invention provides in a further aspect the microorganism IMI 362184 per se and mutants thereof.

Mutants of the IMI 362184 may arise spontaneously or may be produced by a variety of methods including those outlined in Techniques for the Development of Microorganisms by H I Adler in "Radiation and Radioisotopes for Industrial Microorganisms", Proceedings of the Symposium, Vienna 1973, p241, International Atomic Energy authority. Such methods include ionising radiation, chemical methods, e.g. treatment with N-methyl-N'-nitro-N-nitrosoguanidine (NTG), heat, genetic techniques, such as recombination and transformation, and selective techniques for spontaneous mutants.

The preparation of the compound of formula (V) by fermentation may be effected by conventional means i.e. by culturing a suitable organism in the presence of assimilable sources of carbon, nitrogen and mineral salts, and thereafter isolating the desired product.

Assimilable sources of carbon, nitrogen and minerals may be provided by either simple or complex nutrients. Sources of carbon will generally include glucose, maltose, starch, glycerol, molasses, dextrin, lactose, sucrose, fructose, galactose, myo-inositol, D-mannitol, soya bean oil, carboxylic acids, amino acids, glycerides, alcohols, alkanes and vegetable oils. Sources of carbon will generally comprise from 0.5 to 10% by weight of the fermentation medium. Fructose, glucose and sucrose represent preferred sources of carbon.

Sources of nitrogen will generally include soya bean meal, corn steep liquors, distillers solubles, yeast extracts, cottonseed meal, peptones, ground nut meal, malt extract, molasses, casein, amino acid mixtures, ammonia (gas or solution), ammonium salts or nitrates. Urea and other amides may also be used. Sources of nitrogen will generally comprise from 0.1 to 10% by weight of the fermentation medium.

Nutrient mineral salts which may be incorporated into the culture medium include the generally used salts capable of yielding sodium, potassium, ammonium, iron, magnesium, zinc, nickel, cobalt, manganese, vanadium, chromium, calcium, copper, molybdenum, boron, phosphate, sulphate, chloride and carbonate ions.

Cultivation of the organism will generally be effected at a temperature of from 20° to 40⁰C, preferably from 20 to 35⁰C, especially about 25°C, and will desirably take place with aeration and agitation e.g. by shaking or stirring. The medium may initially be inoculated with a small quantity of mycelium and/or spores. The vegetative inoculum obtained may be transferred to the fermentation medium, or to one or more seed stages where further growth takes place before transfer to the principal fermentation medium. The fermentation will generally be carried out in the pH range 3.5 to 9.5, preferably 4.5 to 7.5. It may be necessary to add a base or an acid to the fermentation medium to keep the pH to within the desired range. Suitable bases which may be added include alkali metal hydroxides such as aqueous sodium hydroxide or potassium hydroxide. Suitable acids include mineral acids such as hydrochloric, sulphuric or phosphoric acid.

The fermentation may be carried out for a period of 4-30 days, preferably about 5-15 days. An antifoam may be present to control excessive foaming and added at intervals as required. Carbon and/or nitrogen sources may also be fed into the fermentation medium as required.

The compound of formula (V) is associated mainly with the cells and may be brought into solution either by addition of an acid and a water-miscible organic solvent, or more preferably by addition of a base (e.g. sodium hydroxide). Cells may be separated from these solutions either by centrifugation, conventional filtration or membrane filtration. The liquor may be optionally thereafter treated with an acid such as sulphuric acid until the pH is below 6 (e.g. about pH 4.5).

The compound of formula (V) may be isolated and purified by a variety of fractionation techniques, for example adsorption-elution, precipitation, fractional crystallisation, solvent extraction and liquid-liquid partition which may be combined in various ways.

Adsorption onto a solid support followed by elution has been found to be particularly suitable for isolating and purifying the compound of formula (V).

Suitable solid supports include silica; a non-functional macroreticular adsorption resin, for example cross-linked styrene divinyl benzene polymer resins such as CG161 and Amberlite XAD-2, XAD-4, XAD-16 or XAD-1180 resins (Rohm & Haas Limited) or Kastell S112 (Montedison); a substituted styrene-divinyl benzene polymer such as Diaion SP207 (Mitsubishi); an anion exchanger [e.g. IRA-958 or MacroPrep High Q (BioRad)], an organic solvent-compatible cross-linked dextran such as Sephadex LH20 (Pharmacia UK Limited), or on reverse phase supports such as hydrocarbon linked silica, e.g. C₁₈-linked silica.

The compound of formula (V) may also be isolated and purified by the use of a liquid anion exchanger such as LA 2.

Suitable solvents for the elution of the compound of formula (V) will, of course, depend on the nature of the absorbent. When using a polymer resin such as XAD-16 water-miscible solvents such as methanol, acetone, isopropanol or acetonitrile in various proportions in water may be particularly suitable.

The presence of the compound of formula (V) during the extraction/isolation procedures may be monitored by conventional techniques such as high performance liquid chromatography (HPLC) or UV spectroscopy or by utilising the optical rotation or other property of the compound.

Where the compound of formula (V) is obtained in the form of a solution in an organic solvent, for example after purification by absorption/elution, the solvent may be removed by conventional procedures, e.g. by evaporation, to yield the required compound. If desired, the compound may be further purified by chromatographic techniques such as countercurrent chromatography using a coil extracter such as a multi-layer coil extracter or high performance liquid chromatography or supercritical fluid chromatography on adsorbents such as carbon, alumina, vanadium, polymeric resins or silica, with or without bonded phases. Suitable solvents/eluents for the chromatographic purification/separation of the compound of formula (V) will of course depend on the nature of the adsorbent. When using a C8 bonded silica, mixtures of acetonitrile and water are particularly suitable. Alternatively, the compound may be further purified by solvent extraction, for example using an appropriate organic solvent such as a ketone (e.g. acetone or methyl ethyl ketone), a halogenated hydrocarbon, an alcohol (e.g. methanol), a diol (e.g. propane-1,2-diol or butane-1,3-diol) or an ester (e.g. methyl acetate or ethyl acetate). In a further alternative, solutions of compound (V) may be further purified by treatment with adsorbents that selectively remove impurities when added at appropriate levels (e.g. DEAE-cellulose) or by crystallisation (e.g. from a mixture of acetonitrile and water) or using a combination of the above procedures.

The biotransformation of sordarin to 4'-demethylsordarin, the compound of formula (V), may be effected by incubating sordarin in a culture comprising a suitable organism and sources of carbon and nitrogen, including those sources specified hereinabove, and thereafter isolating the compound of formula (V) from the culture.

Microorganisms capable of demethylating sordarin at the 4'-position may readily be identified by using a small scale test and analysing a test sample obtained using standard methodology, for example, using HPLC. Examples of microorganisms which have been identified as sordarin demethylators include Streptomyces capreolus ATCC 31963, Streptomyces avermitilis ATCC 31272, Streptomyces armentosus NRRL 3176, Streptomyces antibioticus ATCC 31771, Streptomyces rimosus ATCC 23955, Streptomyces platensis ATCC 29778, Streptomyces mashuensis ATCC 23934, Streptomyces eurythermus ATCC 14975, Nocardia orientalis ATCC 43491 and Cunninghamella echinulata var elegans ATCC 36112.

Cultivation of the organism will generally be effected at a temperature of from 20° to 40°C, preferably from 20° to 35°C, especially about 28°C, and will desirably take place with aeration and agitation, e.g. by shaking or stirring. The medium may initially be inoculated with a small quantity of mycelium and/or spores. The vegetative inoculum obtained may be transferred to the fermentation medium or to one or more seed stages where further growth (e.g. over about 1-3 days) takes place before transfer to the principal fermentation medium. The principal fermentation medium will also comprise sordarin and the fermentation will generally be carried out in a pH range of 3.5 to 9.5, preferably 4.5 to 7.5. It may be necessary to add a base or an acid to the fermentation medium to keep the pH to within the desired range. Suitable bases which may be added include alkali metal hydroxides such as aqueous sodium hydroxide or potassium hydroxide. Suitable acids include mineral acids such as hydrochloric, sulphuric or phosphoric acid. Fermentation may be carried out over a period of 2 to 5 days, preferably about 3 days. An antifoam may be present to control excess foaming and added at intervals as required. Carbon and/or nitrogen sources may also be fed into the fermentation medium as required.

The separation and isolation of the compound of formula (V) from the fermentation broth may be effected by the general procedures previously described. When it is desired to lower the pH of the liquor to below pH 6 (e.g. to about pH 2.5) this may conveniently be achieved by the addition of an acid such as orthophosphoric acid.

It will be appreciated that biotransformation may be effected according to a number of alternative methods. For example, cells may be grown and harvested prior to addition to a solution of sordarin in, for example, buffer, spent fermentation medium or water. It is also feasible that the appropriate enzymes could be isolated and used (with appropriate co-enzymes) or the enzymes cloned and over-expressed.

As stated hereinabove, sordarin is a known compound, which may be obtained using procedures described in the relevant art. Thus, for example, the preparation of sordarin by the cultivation of Sordaria araneosa NRRL 3196 (also deposited in the ATCC as ATCC 36386) is described in British Patent Specification No. 1,162,027. Specific examples of the preparation of sordarin using similar procedures are reported hereinafter.

6'-Hydroxysordarin, a compound of formula (VI), may conveniently be prepared under fermentation conditions similar to those described for preparing sordarin using Sordaria araneosa NRRL 3196 or a suitable mutant thereof, and may be isolated by similar general procedures to those described above for the compound of formula (V). For example, broth containing 6'-hydroxysordarin may be treated with base to solubilise the desired compounds and after removal of cells the desired compounds may be adsorbed and eluted from a suitable adsorbent (e.g. XAD-16 or CG161) with a suitable solvent as described above for the compound of formula (V) to provide after concentration a purified extract. The extract may be subjected to preparative HPLC to separate 6'-hydroxysordarin from other products of the fermentation and the appropriate fractions from this chromatography, after batch adsorption and elution or other suitable concentration method, may be subjected to further chromatography to yield substantially pure 6'-hydroxysordarin. It is to be understood that this process for preparing 6'-hydroxysordarin represents a further aspect of the present invention.

The examples hereinafter illustrate aspects of the present invention and are not intended to limit the invention in any way.

### PREPARATION 1

### Production of sordarin

A culture of *Sordaria araneosa* NRRL3196 (ATCC36386) was grown on an agar medium until mature growth occurred. 6mm diameter plugs of the agar containing the growth were transferred to sterile water or Brain Heart Infusion broth (Oxoid) + 10% glycerol and stored at ambient temperature or -140°C respectively. A suspension containing 2 of these agar plugs was used to inoculate a 250ml Erlenmyer flask containing 50ml of medium FS.

| Medium FS | g/L |
|---|---|
| Peptone(Oxoid L34) | 10 |
| Malt extract (Oxoid L39) | 21 |
| Glycerol (Glycerine CP) | 40 |
| Junlon 110 (Honeywell & Stein) | 1 |
| Distilled water | |

The culture was incubated at 25°C for 5 days on a rotary shaker operated at 250 rpm with a 50mm diameter orbital motion. Aliquots (2ml) of the developed inoculum were used to inoculate further 250 ml Erlenmyer flasks containing medium FS (50ml) which were incubated as described above. 80ml of the bulked shake flask developed inoculum was used to inoculate each of two 7L fermenters containing 5L of medium FS. The fermentations were controlled to a temperature of 25C. The culture was agitated at 400 rpm and aerated at 2 Lpm. After 3 days fermentation, 10L of culture was used to inoculate a 780L fermenter containing 500L of medium SM55VAR.

| SM55VAR | g/L |
|---|---|
| Glucidex 32D (Roquette Frere) | 74 |
| Peptone (Oxoid L37) | 10 |
| Proflo (Traders Protein) | 30 |
| Beet molasses | 15 |
| MgSO₄.7H₂O(BDH) | 5 |
| CaCO₃ (BDH) | 5 |
| FeSO₄.7H₂O (Sigma) | 2 |
| ZnSO₄.7H₂0 (BDH) | 0.04 |
| L-trytophan (Sigma) | 2 |
| PPG2000 (K & K Greef) | 0.5 |
| Silicone 1520 (Dow Corning) | 0.04 |
| Distilled water | |

The fermentation was controlled to a temperature of 25°C. The broth was agitated at 300-350 rpm and aerated at 200 Lpm. 70% (w/v) Meritose (Tunnel Refineries) solution was fed to the culture to maintain a positive residual glucose concentration. Distilled water was fed to maintain a culture volume of 500L. PPG2000 antifoam was added on demand to control foaming. Whole broth extracts (in aqueous acetonitrile + 1% trifluoroacetic acid) were assayed for presence of sordarin by reverse phase HPLC. The culture was harvested after 11 days when the extract of a broth sample indicated a sordarin titre of 0.6 g/L. Fermentation broth was made 0.1M with respect to sodium hydroxide and after storage at ambient temperature overnight was filtered through Dicalite on a rotary vacuum filter (1% Dicalite was added to the broth as filter aid). The filtrate was adjusted to pH 6-7 with concentrated sulphuric acid and the solution was applied to XAD16 resin (10 volumes of filtrate/volume resin). The adsorbent was washed with water and acetone:water (1:3) in both cases to give a clear effluent before sordarin was eluted with acetone:water (3:1 ; 2 column volumes collected). Flow rate throughout the process was between 1-2 column volumes/hr. The eluate was concentrated to a small volume (8.5L). The concentrate was adjusted to pH 3 with phosphoric acid and stood overnight at ambient temperature to allow precipitated sordarin to settle. Supernatant was decanted then centrifuged and the supernatant was discarded. Centrifuge pellets and precipitate were taken up in 75% aqueous acetonitrile to give 3.9L of a dark brown solution. To this was added 1.0L 0.2M NH₄H₂PO₄ with stirring, and the solution was adjusted to pH 4.0 with phosphoric acid, to give a final volume of 5.0L with approximate composition 60% acetonitrile - 0.1M NH₄H₂PO₄. The crude sordarin solution (5.0L) from above was subjected to preparative HPLC in 10 injections (450-550ml each) on a column (15cm x 25cm) of 7mm Kromasil C8 in mobile phase of 50% acetonitrile - 0.1M NH₄H₂PO₄, pH 4 (50L acetonitrile made up to 100L with water containing 575g NH₄H₂PO₄ and 40ml H₃PO₄), flow-rate 600ml/min, detection by UV absorbance (λ 210nm). The fraction eluting between 15.4 and 19.2 min was collected. Pooled fractions from the 10 injections (23L) were diluted with water to 50L and this solution was pumped back through the Kromasil column at 28L/h. The column was washed with water (25L) then eluted with 90% acetonitrile (10L). The eluate was evaporated to a residue of 1300ml which was freeze-dried to yield the title compound as a buff powder (105.6g). MS and NMR analysis of the product showed equivalence with an authentic sample of sordarin.

### PREPARATION 2

### Production of sordarin potassium salt Sordaria araneosa NRRL3196 (ATCC36386) was maintained in Brain Heart

Infusion broth (Oxoid) + 10% glycerol at -140°C as described in Preparation 1. A suspension containing 2 agar plugs was used to inoculate a 250ml Erlenmyer flask containing 50ml of medium FS. The culture was incubated at 25°C for 5 days on a rotary shaker operated at 250 rpm with a 50mm diameter orbital motion. Aliquots (2ml) of the developed inoculum were used to inoculate further 250 ml Erlenmyer flasks containing medium FS (50ml) and incubated as described above. 80ml of the bulked shake flask developed inoculum was used to inoculate each of two 7L fermenters containing 5L of medium FS. The fermentations were controlled to a temperature of 25°C. The culture was agitated at 400 rpm and aerated at 2 Lpm. After 3 days fermentation, 10L of culture was used to inoculate a 780L fermenter containing 500L of medium SD1.

| SD1 | g/L |
|---|---|
| Meritose (Tunnel refineries) | 22 |
| Lactose | 20 |
| Glucidex 32D (Roquette Frere) | 30 |
| Arkasoy (The British Arkady Co.) | 20 |
| CSL | 20 |
| FeCl₃.6H₂O | 0.05 |
| NH₄H₂PO₄ | 5 |
| ZnSO₄.7H₂O (BDH) | 0.1 |
| PPG2000 | 0.5 |
| Distilled water | |

The fermentation was controlled to a temperature of 25°C. The broth was agitated at 350 rpm and aerated at 200 Lpm. Distilled water was fed to maintain a culture volume of 500L. Whole broth extracts were assayed for presence of sordarin by reverse phase HPLC. The culture was harvested after 6 days when the extract of a broth sample indicated a sordarin titre of 1.3 g/L. A 50L sample of harvest broth was made 0.1M with respect to sodium hydroxide and stored at 4°C overnight. Cells were removed by vacuum filtration through a bed of Dicalite adding an additional 2% Dicalite to the broth as filter aid. The filtrate was adjusted to approximately pH 6 with concentrated sulphuric acid. Broth extract was pumped through a bed of Amberchrom CG161 resin at 320ml/min (0.64 bed volumes per min). The effluent was monitored by HPLC. After 45 min (equivalent to a pumped volume of 14.4L) sordarin began to break through and pumping was halted. The adsorbent was washed with water (2L) then 25% v/v acetone in water (2L). Sordarin was eluted with 75% v/v acetone in water (1.5L). The 75% v/v acetone eluate was evaporated to about 200ml by rotary evaporation at 40°C. 200ml butan-1-ol was added and the evaporation continued until a viscous oil remained (containing some butan-1-ol). The oily residue was extracted with hot methanol (2 x 500ml). Extracts were combined, filtered (Whatman no 1 paper) then evaporated at 40 to 45°C to give a viscous oil. This was extracted with hot acetone (2 x 500ml). Acetone extracts were combined, filtered and evaporated to a viscous oil. Propan-2-ol (350ml) was added and the oil dissolved at 45°C to give a clear brown solution. A solution of potassium-2-ethylhexanoate (39% w/w solution in propan-2-ol, 54g) was weighed into a 500ml conical flask. The sordarin-containing solution in propan-2-ol was poured into the conical flask, the contents mixed, and left to stand for 4h at room temperature. The solution was seeded with sordarin potassium salt (about 5mg) and the stoppered flask was stored for 3 days at 4°C. The off-white solid which formed was filtered under vacuum (No 4 sinter funnel) and the filter cake was washed with propan-2-ol (about 20-30ml). The solid was transferred to a crystallising dish and dried *in vacuo* over P₂O₅ for 16h to give the title compound (10.5g).

### PREPARATION 3

### Production of sordarin potassium salt

500 litres of culture broth was prepared as in Preparation 1. The broth was made 0.1M with respect to sodium hydroxide and after 4 days at 0°C was filtered through a bed of Dicalite on a rotary vacuum filter. 1% Dicalite was added to the broth as filter aid. The pH of the filtrate was adjusted from pH 9.6 to pH 7.5 with concentrated sulphuric acid. Filtrate (10L) was adjusted to pH 6 with H₃PO₄ and applied to a bed (200ml) of XAD-16 resin packed in water at a flow-rate of 1-1.5 bed volumes/h. The resin bed was washed with water (1 bed volume) then with 25% aqueous isopropanol (2.5 bed volumes) before elution with neat isopropanol. After a fore-run of 50ml the isopropanol eluate was collected as 100ml fractions. Sordarin-rich fractions 2-6 inclusive were pooled and evaporated to half volume. Isopropanol was added to bring the volume back to 500ml, then the evaporation to half volume repeated to remove residual water by azeotropic distillation. After a third evaporation to half volume the residue was filtered and the filter was washed with isopropanol. To the combined filtrate and washings (400ml) was added a solution of potassium 2-ethylhexanoate (8g) in isopropanol (100ml). The mixture was seeded with sordarin potassium salt and stood for several days at 4°C whilst a slow crystallisation took place. Crystals were filtered on a No. 3 sinter, washed with a little ice-cold isopropanol and dried *in vacuo* to yield the title compound as a pale brown powder (4.85g).

### PREPARATION 4

### Production of 4'-demethylsordarin

(i) IMI 362184 was maintained in Brain Heart Infusion broth (Oxoid) + 10% glycerol at -140°C as described in Preparation 1. A suspension containing 2 agar plugs was used to inoculate a 250ml Erlenmyer flask containing 50ml of medium FS. The culture was incubated at 25°C for 5 days on a rotary shaker operated at 250 rpm with a 50mm diameter orbital motion. Aliquots (2ml) of the developed inoculum were used to inoculate further 250 ml Erlenmyer flasks containing medium FS (50ml) and incubated as described above. 80ml of the bulked shake flask developed inoculum was used to inoculate each of two 7L fermenters containing 5L of medium FS. The fermentations were controlled to a temperature of 25°C. The culture was agitated at 400 rpm and aerated at 2 Lpm. After 3 days fermentation, 10L of culture was used to inoculate a 780L fermenter containing 500L of medium SM55VAR (as described in Preparation 1). The fermentation was controlled to a temperature of 25°C. The broth was agitated at 350 rpm and aerated at 500 Lpm. 70% w/v Meritose (Tunnel Refineries) solution was fed to the culture to maintain a positive residual glucose concentration. Distilled water was fed to maintain a culture volume of 500L. Whole broth extracts were assayed for presence of 4'-demethylsordarin by reverse phase HPLC. The culture was harvested after 10 days when the extract of a broth sample indicated a 4'-demethylsordarin titre of 0.8 g/L. Fermentation broth was made 0.1M with respect to sodium hydroxide and after 1 hour at ambient temperature was filtered through Dicalite on a rotary vacuum filter (1% Dicalite was added to the broth as filter aid). The filtrate was adjusted to pH 4.5 with concentrated sulphuric acid and the solution was applied to XAD16 resin (20g product/L resin). The adsorbent was washed with 0.1% phosphoric acid (10 column volumes) and acetonitrile:water 1:4 (6 column volumes) before the product was eluted with acetonitrile:water (1:1 ; 2 column volumes). Flow rate throughout the process was between 1-2 column volumes/hr. The eluate was concentrated to dryness with the addition of butan-1-ol and the solid was extracted with methanol (12L) followed by acetone (10L) at 60°C. Insoluble material was removed at each stage by filtration through a no 3 glass sinter and the extracts concentrated to dryness as before. The solid was crystallised from acetonitrile:water (3:7) before being recrystallised from the same solvent and dried to constant weight over P₂O₅ to give the title compound (244.0g), which by proton NMR analysis showed equivalence with an authentic sample of 4'-demethylsordarin.
(ii) The fermentation procedure in (i) above was followed, and after the broth was made 0.1M with respect to sodium hydroxide this was ultrafiltered through ETNA 10A membrane (10kDalton cut-off). After diafiltration with water the bulked permeate was a clear solution. After adjustment to pH 5.2, using concentrated sulphuric acid, the permeate was loaded to a column of XAD16 resin at a rate of 2 column volumes per hour to give a final loading of 32g 4'-demethylsordarin per litre of resin. The column was washed at a rate of 2 column volumes per hour, first with 0.1% v/v phosphoric acid and then with 20% v/v acetonitrile/water (10 column volumes of each). The column was eluted with 65% acetonitrile/water at 2 column volumes per hour. A forerun of 0.75 column volumes was discarded. 85% of the loaded 4'-demethylsordarin was recovered in the next 1.6 column volumes of eluate. The rich eluate was treated by stirring for 5 minutes with 2% w/v of DE52 cellulose, which was removed by filtration. An aliquot of DE52 treated eluate was concentrated to 62% of the original volume (43% acetonitrile) using a rotary evaporator. The concentrated eluate was held at 4°C for 15 hours to crystallise and the solids formed collected by filtration through a glass sinter. The crystals were washed with four cake volumes of 30% v/v acetonitrile/water and dried at 30°C in vacuo. The title compound (1.94g) was recovered from the concentrated eluate as a pale grey solid.

### PREPARATION 5

### Screen for microorganisms capable of demethylating sordarin at the 4' position

Microorganisms capable of demethylating sordarin at the 4'-position could be identified by growing them at 28°C (bacteria) or 25°C (fungi) in 10ml volumes of SB1 (bacteria) or FB1 (fungi) in 50ml conical flasks shaken at 250rpm. After 2 days, sordarin was added to a final concentration of 0.5mg/ml (from a 200mg/ml stock solution in 80% ethanol) and the flasks were then incubated for a further 3 days. A 500µl sample of whole culture was mixed in an Eppendorf tube with 500µl of 80% acetonitrile/2% trifluoroacetic acid and left to extract at room temperature for 30 minutes. The extract supernatant, obtained by centrifuging samples in a microfuge was assayed by isocratic HPLC for the presence of 4'-demethylsordarin. 4'-demethyl sordarin eluted at 3.35mins with a mobile phase of 35% acetonitrile in water, flow rate 2ml/min, using a Spherisorb C₆ column (5µm, 15cm x 4.6mm). By this method, the following microorganisms were identified as sordarin demethylators:

| | |
|---|---|
| *Streptomyces capreolus* | ATCC 31963 |
| *Streptomyces avermitilis* | ATCC 31272 |
| *Streptomyces armentosus* | NRRL 3176 |
| *Streptomyces antibioticus* | ATCC 31771 |
| *Streptomyces rimosus* | ATCC 23955 |
| *Streptomcyes platensis* | ATCC 29778 |
| *Streptomyces mashuensis* | ATCC 23934 |
| *Streptomyces eurythermus* | ATCC 14975 |
| *Nocardia orientalis* | ATCC 43491 |
| *Cunninghamella echinulata var elegans* | ATCC 36112 |

| SB1 Medium | g/L |
|---|---|
| Arkasoy | 25 |
| Yeast extract | 5 |
| KH₂PO₄ | 5 |
| Glucose | 20 |
| Distilled Water pH 7 | |

| FB1 Medium | |
|---|---|
| Soya oil | 30 |
| Arkasoy | 10 |
| Yeast extract | 5 |
| K₂HPO₄ | 5 |
| Glucose | 20 |
| Distilled Water pH 5.5 | |

### PREPARATION 6

### Production of 4'-demethylsordarin by biotransformation of sordarin

0.3ml of a spore suspension of *Streptomyces capreolus* ATCC 31963 (in 15% v/v glycerol stored at -20°C) was inoculated into 30ml SB1 medium in a 250ml Erlenmeyer flask to give a seed culture which was incubated at 28°C and 250rpm on a rotary shaker. After 4 days, 0.5ml of this was used to inoculate 35ml SB1 in a 250ml flask which was grown for 48 hours at 28°C, 250rpm. At this stage, the culture was aliquoted as 10ml amounts into 50ml Erlenmeyer flasks which were fed with 5mg sordarin (from a 200mg/ml stock solution in ethanol). Incubation was continued for a further 3 days. 80% v/v acetonitrile in water (14ml) was added to whole broth (14ml) and the mixture was kept at room temperature and occasionally agitated. After 30 minutes, the cells were removed by centrifugation. Acetonitrile was removed by evaporation and the pH of the aqueous solution was adjusted to 2.5 with orthophosphoric acid. The solution was passed through a column containing Amberlite XAD-16 resin (bed volume 5ml). The adsorbent was washed sequentially with water (10ml), 10% v/v acetonitrile in water (20ml), 30% v/v acetonitrile in water (10ml), 50% v/v acetonitrile in water (20ml) and 90% v/v acetonitrile in water (10ml). Fractions were monitored by HPLC; 4'-demethylsordarin was located in the 50% v/v acetonitrile in water eluate. The fraction containing 4'-demethylsordarin was evaporated to dryness *in vacuo* at the room temperature and the residue was re-dissolved in 35% v/v acetonitrile in water (15ml). 4'-demethylsordarin was purified by preparative HPLC:

- Column: Spherisorb 5 micron C₆ 25cm x 2.5cm
- Flow rate: 25ml/min
- Detection: UV at 210nm
- Mobile Phase: 350ml acetonitrile made up to 1000ml with 0.05M ammonium dihydrogen phosphate in water. pH adjusted to 2.5 with orthophosphoric acid
- Injection volume: 4.5ml

4'-Demethylsordarin was eluted after 10.0 minutes under these conditions. The pooled fractions from four HPLC runs were diluted 1:1 with water then pumped back onto the silica (after washing this with acetonitrile and re-equilibrating with water). The adsorbent was washed with water (200ml) and adsorbed product was eluted with 95% v/v acetonitrile in water (200ml). The acetonitrile/water eluate was evaporated to remove organic solvent, and the aqueous solution freeze dried to yield 4'-demethylsordarin (1.5mg) as a white powder. δ (¹H,CDCl₃); 9.74(s,1H); 6.08(brd,3,1H); 4.70(d,1.5,1H); 4.16(d,9.5,1H); 4.08(dd,4.5,3.5,1H); 3.88(dd,4.5,1.5,1H); 3.75(dq,8.5,6,1H); 3.62(d,9.5,1H); 3.68(dd,8.5,3.5,1H); 2.65(m,1H); 2.34(m,1H); 1.32(d,6,3H); 1.30(d,12.5,1H); 1.23(m,1H); 1.04(d,7,3H); 0.99(d,7,3H); 0.81(d,7,3H)

### INTERMEDIATE 1

### [1R-(1α, 3aβ, 4β, 4aβ, 7β, 7aα, 8aβ)] 8a-[(6-deoxy-4-O-methyl-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A solution of sordarin (10.0g) in dichloromethane (150ml) was treated dropwise with a solution of diphenyldiazomethane in dichloromethane (0.35M, 85ml). The resulting solution was stirred at room temperature for 24 hours. The solvent was removed under reduced pressure and the residue was purified by flash column chromatography on silica gel eluting with hexane:ethyl acetate (4:1) and (2:1). The fractions were combined and evaporated to yield the title compound (11.8g) as a white foam.
δ (¹H, CDCl₃): 10.00 (s, 1H, CHO), 7.63 (m, 10H, 2Ph), 7.26 (s, 1H, CHPh₂), 6.30 (dd, 1H, H-2, J=1.2 and 3.3 Hz), 4.92 (d, 1H, H-1', J=0.9 Hz), 4.84 (t, 1H, H-3', J=3.3 Hz),4.03 and 4.35 (2d, 2H, 8aCH₂, J= 9 Hz), 3.88 (m, 1H, H-2'), 3.70 (dq, 1H, H-5', J=6.3 and 9.3 Hz), 3.41 (s, 3H, 4'-OMe), 3.20 (dd, 1H, H-4', J=3.3 and 9 Hz), 2.75 (t, 1H, H-1).

### INTERMEDIATE 2

(a) [1R-(1α, 3aβ, 4β, 4aβ, 7β, 7aα, 8aβ)] 8a-[(6-Deoxy-4-*O*-methyl-2,3-di-*O*-tosyl-β-D-altropranosyloxy)methyl]-4-formyl-4,4a,5,6,7,7a,8,8a-octahydro-7- methyl-3-(1-methylethyl)-1,4-methano-*s*-indacene-3a(1H)-carboxylic acid, diphenylmethyl ester and
(b) [1R-(1α, 3aβ, 4β, 4aβ, 7β, 7aα, 8aβ)] 8a-[(6-Deoxy-4-*O*-methyl-2-*O*-tosyl-β-D-altropyranosyloxy)methyl]-4-formyl-4,4a,5,6,7,7a,8,8a-octahydro-7-methyl-3-(1-methylethyl)-1,4-methano-*s*-indacene-3a(1H)-carboxylic acid, diphenylmethyl ester
Intermediate 1 (2g) and 4-dimethylaminopyridine (500mg) were dissolved in dry pyridine (30ml). A solution of tosyl chloride (960mg) in dry pyridine (20ml) was added dropwise. The reaction mixture was stirred at room temperature for 4 days. The solvent was evaporated in vacuo to give a yellow residue, which was chromatographed on silica gel column, eluting with hexane:ethyl acetate (4:1) and (1:1) to afford title compound (a) with the higher Rf. as a white foam (1.65g) and title compound (b) with the lower Rf. also obtained as a white foam (1.17g).
(a) δ (¹H, CDCl₃): 9.71 (s, 1H, CHO), 7.85-7.91 (m, 4H, ortho-Ts), 7.26-7.41 (m, 14H, meta-Ts and 2 Ph), 6.95 (s, 1H, CO₂CHPh₂), 5.87 (dd, 1H, H-2, J=1.2 and 3.3 Hz), 4.92 (dd, 1H, H-3', J=3.3 and 4.2 Hz), 4.65 (dd, 1H, H-2', J=1.2 and 4.5 Hz), 4.61 (d, 1H, H-1', J=1.2 Hz), 3.50 and 3.94 (2d, 2H, 8aCH₂, J=9 Hz), 3.68 (dq, 1H, H-5', J=6.6 and 9 Hz), 3.16 (dd, 1H, H-4', J=3 and 9Hz), 2.78 (s, 3H, 4'-OMe), 2.45 and 2.47 (2s, 6H, 2Ts).
(b) δ (¹H, CDCl₃): 9.71 (s, 1H, CHO), 7.85 (m, 2H, ortho-Ts), 7.26-7.41 (m, 12H, meta-Ts and 2Ph), 6.95 (s, 1H, CHPh₂), 5.87 (dd, 1H, H-2, J=1.5 and 3.3 Hz), 4.61 (d, 1H, H-1', J=1.5 Hz), 4.55 (dd, 1H, H-2',J=1.2 and 4.2 Hz), 4.37 (m, 1H, H-3'), 3.50 and 3.95 (2d, 2H, 8aCH₂, J= 9Hz), 3.70 (dq, 1H, H-5', J=8.4 and 6.3 Hz), 3.23 (dd, 1H, H-4',J=3.3 and 8.7 Hz), 2.42 (s, 3H, Ts).

### INTERMEDIATE 3

### [1R-(1α, 3aβ, 4β, 4aβ, 7β, 7aα, 8aβ)] 8a-[(2,3-Anhydro-6-deoxy-4-O-methyl-β-D-mannopyranosyloxy) methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

Sodium (0.5g) was added to anhydrous methanol (50ml) with cooling at 0^{o}C, followed by Intermediate 2(a) (670mg) in dry dichloromethane (20ml). The mixture was stirred for 5 days at room temperature and 4 days at reflux. The mixture was filtered off and the solvent removed. The residue was purified by flash chromatography eluting with hexane:ethyl acetate (3:1) to afford the title compound (330mg).
δ (¹H, CDCl₃): 9.75 (s, 1H, CHO), 7.26-7.44 (m, 10H, 2Ph), 6.99 (s, 1H, CO₂CHPh₂), 6.08 (dd, 1H, H-2, J=1.5 and 3 Hz), 4.68 (s, 1H, H-1'), 3.78 and 4.11 (2d, 2H, 8aCH₂, J=9 Hz), 3.49 (s, 3H, 4'-OMe), 3.26 (d, 1H, H-2', J=3.9 Hz), 3.18 (m, 1H, H-5'), 3.12 (d, 1H, H-3', J=3.6 Hz), 3.08 (m, 1H, H-4'), 2.86 (m, 1H, H-1').

### INTERMEDIATE 4

### [1R-(1α, 3aβ, 4β, 4aβ, 7β, 7aα, 8aβ)] 8a-[(2,3-Anhydro-6-deoxy-4-O-methyl-β-D-allopyranosyloxy) methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

Dry sodium hydride (25.9mg) was suspended in dry dimethylformamide (5ml). Intermediate 2(b) (470mg) in dry dimethylformamide was added dropwise and the mixture stirred for 40 minutes at room temperature. The reaction mixture was poured with stirring into ice-cold water (20ml) and the resulting solution was treated with ethyl acetate (3x20ml). The organic layers were combined and dried over anhydrous magnesium sulphate. The gummy residue obtained upon evaporation of solvents was purified by flash column chromatrography eluting with hexane:ethyl acetate (5:1) to yield the title compound as a white foam (332mg).
δ (¹H, CDCl₃): 9.73 (s, 1H, CHO), 7.26-7.44 (m, 10H, 2Ph), 6.98 (s, 1H, CO₂CHPh₂), 6.08 (dd, 1H, H-2, J=1.5 and 3 Hz), 4.60 (s, 1H, H-1'), 3.76 and 4.03 (2d, 2H, 8aCH₂, J=9.3 Hz), 3.51 (m, 4H, H-4' and 4'-OMe), 3.33 (m, 3H, H-2', H-3' and H-5'), 2.80 (m, 1H, H-1).

### INTERMEDIATE 5

### [1R-(1α, 3aβ, 4β, 4aβ, 7β, 7aα, 8aβ)] 8a-[(2,3-Anhydro-6-deoxy-4-O-methyl-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 4 (100mg) in ethyl acetate (15ml) was added 10% palladium on charcoal (100mg) under nitrogen. The mixture was shaken in a Parr apparatus under 15 psi hydrogen for 1 hour at room temperature. The catalyst was filtered off and the solvent evaporated to dryness. The residue was purified by flash chromatography on silica gel, eluting with hexane:ethyl acetate (3:1) and dichloromethane: methanol (15:1). The appropiate fractions were combined and the solvent removed to give the title compound (70mg) as a foam.
δ (¹H, CDCl₃): 9.77 (s, 1H, CHO), 6.09 (dd, 1H, H-2, J=1.2 and 3.6 Hz), 4.67 (d, 1H, H-1', J=0.9 Hz), 3.58 and 4.17 (2d, 2H, 8aCH₂, J=9.6 Hz), 3.51 (m, 4H, H-3' and 4'-OMe), 3.38 (m, 1H, H-5'), 3.34 (d, 1H, H-2', J=4.2 Hz), 3.30 (dd, 1H, H-4', J=9 and 1.5 Hz), 2.65 (t, 1H, H-1, J=3.9 Hz).

### INTERMEDIATE 6

### [1R-(1α, 3aβ, 4β, 4aβ, 7β, 7aα, 8aβ)] 8a-[(6-Deoxy-β -D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a solution of 4'-demethylsordarin (10g) in methanol (200ml) was added dropwise a 0.35M solution of diphenyldiazomethane in methylene chloride (90ml, 30mmol) at room temperature, and the mixture was stirred for 6 hours. The solvent was evaporated to dryness and the residue chromatographed in a silica gel flash column with hexane:ethyl acetate (3:1) as eluent to give the title compound (12.6g) as a pale yellow foam.
δ (¹H, CDCl₃): 9.73 (s, 1H, CHO), 6.98 (s, 1H, CHPh₂), 6.05 (dd, 1H, H-2, J=1.5 and 3.6 Hz), 4.65 (d, 1H, H-1', J=1.5 Hz), 4.09, 3.76 (2d, 2H, 8a-CH₂, J=9Hz), 4.01 (m, 1H, H-2'), 3.84 (m, 1H, H-3'), 3.75 (m, 1H, H-5'), 3.69 (m, 1H, H-4'), 2.73 (t, 1H, H-1, J=4.2 Hz).

### INTERMEDIATE 7

### [1R-(1α, 3aβ, 4β, 4aβ, 7β, 7aα, 8aβ)] 8a-[(4-O-Allyl-6-deoxy-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A suspension of Intermediate 6 (2g) and dibutyltin oxide (870mg) in dry toluene (25ml) was refluxed for 2 h in a flask fitted with a Dean-Stark condenser, under nitrogen, and then allowed to stand at room temperature. Allyl bromide (0.3ml) and a 1M solution of tetrabutylammonium fluoride (3.5ml) were added consecutively and the mixture was heated at 40^{o}C for 24 h under nitrogen. The solvent was evaporated to dryness and the residue chromatographed on a silica gel flash column eluting with hexane:acetone (10:1) and (9:1) to give the title compound (1.7g) as a white foam.
δ (¹H, CDCl₃): 9.73 (s, 1H, CHO), 6.98 (s, 1H, CHPh₂), 6.05 (dd, 1H, H-2, J=1.5 and 3.6 Hz), 5.89 (m, 1H, CH=CH₂), 5.25 (m, 2H, CH=CH₂), 4.64 (d, 1H, H-1', J=1.5 Hz), 4.15 (t, 1H, H-3', J=3.3 Hz), 4.09 (m, 3H, CH₂-CH=CH₂ and 8a-CH₂), 3.87 (m, 1H, H-2'), 3.74 (m, 2H, H-5' and 8a-CH₂), 3.38 (dd, 1H, H-4', J=3.3 and 9 Hz), 2.74 (t, 1H, H-1, J=3.9 Hz).

### INTERMEDIATE 8

### [1R-(1α, 3aβ, 4β, 4aβ, 7β, 7aα, 8aβ)] 8a-[(4-O-Benzyl-6-deoxy-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A vigorous stirred mixture of Intermediate 6 (6.0g) and dibutyltin oxide (3.55g) in dry toluene (80ml) was refluxed for 3 hours in a flask fitted with a Dean-Stark condenser under nitrogen atmosphere. The reaction mixture was cooled to 0^{o}C and then treated with benzyl bromide (1.2ml) and tetrabutylammonium fluoride (1M in tetrahydrofuran, 10ml). After heating at 40^{o}C for 18 hours under nitrogen, the reaction was concentrated under reduced pressure and the residue purified by flash chromatography, eluting with hexane:ethyl acetate (6:1) and (4:1). The appropiate fractions were combined and the solvents evaporated to give the title compound (3.73g) as a white foam.
δ (¹H, CDCl₃): 9.73 (s, 1H, CHO), 7.24-7.44 (m, 15H, 3Ph), 6.98 (s, 1H, CO₂CHPh₂), 6.05 (dd, 1H, H-2, J=1.5 and 3.6 Hz), 4.65 (d, 1H, H-1', J=1.2 Hz), 4.57 (AB system, 4'OCH₂Ph, J=11.1 Hz), 4.21 (m, 1H, H-3'), 3.76 and 4.07 (2d, 2H, 8aCH₂, J=9 Hz), 3.87 (m, 1H, H-2'), 3.76 (m, 1H, H-5'), 3.49 (dd, 1H, H-4', J=3.3 and 9 Hz), 2.74 (t, 1H, H-1, J=3.6 Hz).

### INTERMEDIATE 9

### [1R-(1α, 3aβ, 4β, 4aβ, 7β, 7aα, 8aβ)] 8a-[(2-O-Benzyloxycarbonyl-6-deoxy-4-O-methyl-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a solution of intermediate 1 (3 mmol) in dry dichloromethane (15ml) at 0^{o}C under nitrogen atmosphere was added 4-dimethylaminopyridine (6.3mmol). After stirring for 15 minutes, the mixture was cooled to -20^{o}C and a solution of benzylchloroformate (3.6 mmol) in dry dichloromethane (15ml) was added dropwise. After two hours, the solvent was evaporated and the residue purified by flash chromatography using hexane:ethyl acetate (3:1) as eluent to give the title compound (1.2g).
δ (¹H, CDCl₃): 9.70 (s, 1H, CHO), 7.35 (m, 15H, 3xPh), 6.96 (s, 1H, CHPh₂), 5.99 (dd, 1H, H-2, J=1.5 and 3.6 Hz), 5.18 (AB system, 2H, OCH₂Ph, J=12 Hz), 5.00 (dd, 1H, H-2', J=1.5 and 4.2 Hz), 4.70 (d, 1H, H-1', J=1.8 Hz), 4.13 (m, 1H, H-3'), 4.01 and 3.66 (d,d, 1H, 1H, 8a-CH₂, J=9 Hz), 3.75 (m, 1H, H-5'), 3.40 (s, 3H, OMe), 3.17 (dd, 1H, H-4', J=3.3 and 8.4 Hz), 2.62 (t, 1H, H-1, J=3.6 Hz), 2.43 (d, 1H, OH, J=2.4 Hz), 2.21 (m, 1H, CH(CH₃)₂).

### INTERMEDIATE 10

### [1R-(1α, 3aβ, 4β, 4aβ, 7β, 7aα, 8aβ)] 8a-[(4-O-Allyl-2-O-benzyloxycarbonyl-6-deoxy-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a solution of Intermediate 7 (1.4g) in dry methylene chloride (30ml) at 0^{o}C was added dimethylaminopyridine (513mg) and the mixture was stirred for 15 minutes. Benzyl chloroformate (0.35ml) in dry methylene chloride (15ml) was then added dropwise for 15 minutes, and the mixture stirred for 4 h. The mixture was then diluted with methanol (1ml) and methylene chloride (20ml) and washed with water, 1N hydrochloric acid and brine. The organic layer was dried over anhydrous magnesium sulphate, filtered and the solvent evaporated to dryness. The residue was purified by flash column chromatography eluting with hexane:ethyl acetate (7:1) and (5:1) to give the title compound (1.1g).
δ (¹H, CDCl₃): 9.70 (s, 1H, CHO), 6.96 (s, 1H, CHPh₂), 5.99 (dd, 1H, H-2, J=1.5 and 3.6 Hz), 5.88 (m, 1H, CH=CH₂), 5.20 (m, 4H, Ph-CH₂-O, CH₂-CH=CH₂), 4.99 (dd, 1H, H-2', J=1.5 and 4.2 Hz), 4.70 (d, 1H, H-1', J=1.5 Hz), 4.12 (m, 1H, H-3'), 4.03 (m, 3H, CH₂-CH=CH₂ and H-8a-CH₂), 3.76 (m, 1H, H-3'), 3.64 (d, 1H, 8a-CH₂, 8.7 Hz), 3.34 (dd, 1H, H-4', J=3.3 and 8.7 Hz), 2.61 (t, 1H, H-1, J=3.9 Hz).

### INTERMEDIATE 11

### [1R-(1α, 3aβ, 4β, 4aβ, 7β, 7aα, 8aβ)] 8a-[(4-O-Benzyl-2-O-benzyloxycarbonyl-6-deoxy-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a solution of Intermediate 8 (3.7g) in dry dichloromethane (25ml) at 0^{o}C under nitrogen atmosphere was added 4-dimethylaminopyridine (1.85g). After stirring for 15 minutes, the mixture was cooled to -20^{o}C and a solution of benzylchloroformate (0.68ml) in dry dichloromethane (10ml) was added dropwise until the product was detected by tlc (hexane:ethyl acetate 4:1). The solvent was then evaporated to dryness and the residue was purified by flash chromatography using hexane:ethyl acetate (6:1) as eluent to give the title compound (3.6g) as a white foam.
δ (¹H, CDCl₃): 9.70 (s, 1H, CHO), 7.26-7.42 (m, 20H, 4Ph), 6.96 (s, 1H, CO₂CHPh₂), 5.99 (dd, 1H, H-2, J=1.2 and 3.3 Hz), 5.18 (AB system, 2'-OCO₂CH₂Ph, J= 12.3 Hz), 5.00 (dd, 1H, H-2', J=1.5 and 4.2 Hz), 4.71 (d, 1H, H-1', J=1.5 Hz), 4.56 (AB system, 4-OCH₂Ph, J=11.1 Hz), 4.15 (m, 1H, H-3'), 3.66 and 4.00 (2d, 2H, 8aCH₂, J=9 Hz), 3.81 (dq, 1H, H-5', J=6.6 and 9 Hz), 3.43 (dd, 1H, H-4', J=3.3 and 9 Hz), 2.60 (t, 1H, H-1, J=3.6 Hz).

### INTERMEDIATE 12

### [1R-(1α, 3aβ, 4β, 4aβ, 7β, 7aα, 8aβ)] 8a-[(2-O-Benzyloxycarbonyl-6-deoxy-4-O-methyl-3-O-tosyl-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A solution of Intermediate 9 (3 mmol) and 4-dimethylaminopyridine (5.1 mmol) in dry dichloromethane (60ml) was treated dropwise with a solution of tosyl chloride (4.5 mmol) in dry dichloromethane (20ml). After 24 hours, the solvent was evaporated and the residue purified by flash chromatography using hexane:ethyl acetate (5:1) as eluent to give the title compound (1.8g).
δ (¹H, CDCl₃): 9.70 (s, 1H, CHO), 7.84 and 7.32 (d, m, 2H, 17H, Ar), 6.96 (s, 1H, CHPh₂), 5.94 (dd, 1H, H-2, J=1.5 and 3.6 Hz), 5.17 (AB system, 2H, OCH₂Ph, J=12 Hz), 5.10 (m, 1H, H-3'), 4.88 (dd, 1H, H-2', J=3 and 5.4 Hz), 4.68 (d, 1H, H-1', J=2.1 Hz), 3.97 and 3.63 (2d, 2H, 8a-CH₂, J=9 Hz), 3.76 (dd, 1H, H-5', J=6.3 and 8.1 Hz), 3.18 (dd, 1H, H-4', J=3 and 8.1 Hz), 3.05 (s, 3H, OMe), 2.51 (t, 1H, H-1, J=3.6 Hz), 2.43 (s, 3H, CH₃Ar), 2.01 (m, 1H, CH(CH₃)₂).

### INTERMEDIATE 13

### 1R-(1α, 3aβ, 4β, 4aβ, 7β, 7aα, 8aβ)] 8a-[(4-O-Allyl-2-O-benzyloxycarbonyl-6-deoxy-3-O-tosyl-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a solution of Intermediate 10 (1.1g) and dimethylaminopyridine (300mg) in dry methylene chloride (40ml) was added dropwise a solution of tosyl chloride (400mg) in dry methylene chloride (20ml) and the reaction mixture was stirred for 3 days at room temperature. The solvent was evaporated to dryness and the residue chromatographed on a silica gel flash column eluting with hexane:ethyl acetate (9:1) and (6:1) to give the title compound (820mg).
δ (¹H, CDCl₃): 9.70 (s, 1H, CHO), 6.95 (s, 1H, CHPh₂), 5.94 (dd, 1H, H-2, J=1.5 and 3.9 Hz), 5.61 (m, 1H, CH=CH₂), 5.12 (m, 4H, Ph-CH₂-O, CH₂-CH=CH₂), 4.84 (dd, 1H, H-2', J=3 and 5.1 Hz), 4.69 (d, 1H, H-1', J=1.8 Hz), 3.96 and 3.63 (2d, 2H, 8a-CH₂, J=9.0 Hz), 3.76 (m, 4H, H-3', H-5', CH₂-CH=CH₂), 3.38 (dd, 1H, H-4', J=3 and 8.1 Hz), 2.50 (t, 1H, H-1, J=3.9 Hz), 2.42 (s, 3H, CH₃-Ph).

### INTERMEDIATE 14

### [1R-(1α, 3aβ, 4β, 4aβ, 7β, 7aα, 8aβ)] 8a-[(4-O-Benzyl-2-O-benzyloxycarbonyl-6-deoxy-3-O-tosyl-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a solution of Intermediate 11 (3.5g) and dimethylaminopyridine (1.3g) in dry methylene chloride (80ml) was added dropwise a solution of tosyl chloride (2g) in dry methylene chloride (50ml) and the mixture was stirred for 3 days at room temperature. The solvent was evaporated and the residue chromatographed on a silica gel flash column using hexane:ethyl acetate (9:1) as eluent to give the title compound (3.14g).
δ (¹H, CDCl₃): 9.70 (s, 1H, CHO), 6.95 (s, 1H, CHPh₂), 5.95 (dd, 1H, H-2, J=1.2 and 3.3 Hz), 5.16 (m, 3H, Ph-CH₂-O, H-2'), 4.93 (dd, 1H, H-3', J=3 and 4.8 Hz), 4.71 (d, 1H, H-1', J=1.8 Hz), 4.25 (AB system, 2H, O-CH₂Ph, J=11.5 Hz), 3.96, 3.63 (2d, 2H, 8a-CH₂, J=9.0 Hz), 3.83 (m, 1H, H-5'), 3.45 (dd, 1H, H-4', J= 3 and 8.4 Hz), 2.51 (t, 1H, H-1, J=3.9 Hz), 2.35 (s, 3H, CH₃-Ph).

### INTERMEDIATE 15

### [1R-(1α, 3aβ, 4β, 4aβ, 7β, 7aα, 8aβ)] 8a-[(4-0-Benzyl-6-deoxy-3-O-tosyl-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 14 (1.0g) in ethyl acetate (70ml) was added 10% palladium on charcoal (600mg) under nitrogen. The mixture was shaken in a Parr apparatus under 15 psi of hydrogen for 4 hours at room temperature. The catalyst was filtered off and the solvent evaporated to dryness. The residue was purified by flash chromatography on silica gel, eluting with hexane:ethyl acetate (3:1) and (1:1). The appropiate fractions were combined and the solvent was evaporated to give the title compound (530mg).
δ (¹H, CDCl₃): 9.70 (s, 1H, CHO), 7.81 and 7.10-7.30 (2m, 9H, Ts and Ph), 6.08 (dd, 1H, H-2, J=1.5 and 3.3 Hz), 4.91 (dd, 1H, H-3', J=3.3 and 4.5 Hz), 4.68 (d, 1H, H-1', J=1.5 Hz), 4.12 (m, 3H, CH₂Ph and H-2'), 4.06 and 3.64 (2d, 2H, 8aCH₂, J=9.3 Hz), 3.46 (m, 1H, H-4'), 2.65 (m, 1H, H-1), 2.36 (s, 3H, Ts).

### INTERMEDIATE 16

### [1R-(1α, 3aβ, 4β, 4aβ, 7β, 7aα, 8aβ)] 8a-[(6-Deoxy-4-O-methyl-3-O-tosyl-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a solution of Intermediate 12 (1.9mmol) in ethyl acetate (60ml) was added 10% palladium on charcoal (100mg) under nitrogen. The mixture was shaken in a Parr apparatus under 15 psi of hydrogen for 1 hour at room temperature. The catalyst was filtered off and the solution was treated with a 0.35M solution of diphenyldiazomethane (20ml). After 2 hours, the solvent was evaporated and the residue purified by flash chromatography using hexane:ethyl acetate (3:1) as eluent to give the title compound (1.3g).
δ (¹H, CDCl₃): 9.72 (s, 1H, CHO), 7.85 and 7.32 (d, m, 2H, 12H, Ar), 6.98 (s, 1H, CHPh₂), 6.06 (dd, 1H, H-2, J= 1.2 and 3.3 Hz), 4.84 (dd, 1H, H-3', J=3 and 4.2 Hz), 4.64 (d, 1H, H-1', J=1.5 Hz), 4.06 and 3.75 (2d, 2H, 8a-CH₂, J=9.3 Hz), 4.02 (m, 1H, H-2'), 3.70 (m, 1H, H-5'), 3.16 (dd, 1H, H-4', J=3 and 9 Hz), 2.93 (s, 3H, OMe), 2.69 (t, 1H, H-1, J=3.9 Hz), 2.45 (s, 3H, CH₃Ar), 2.30 (d, 1H, OH, J=3 Hz), 2.23 (m, 1H, CH(CH₃)₂).

### INTERMEDIATE 17

### 1R-(1α, 3aβ, 4β, 4aβ, 7β, 7aα, 8aβ)] 8a-[(6-Deoxy-4-O-propyl-3-O-tosyl-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a solution of Intermediate 13 (800mg) in ethyl acetate (200ml) was added 10% palladium on charcoal (300mg) under nitrogen. The mixture was shaken in a Parr apparatus under 20 psi of hydrogen for 1 hour at room temperature. The catalyst was filtered off and the solvent evaporated to dryness. The residue was dissolved in methylene chloride (50ml) and a 0.35M solution of diphenyldiazomethane (6ml) was added dropwise. The mixture was stirred at room temperature for 3 hours. The solvent was evaporated and the residue chromatographed on a silica gel flash column eluting with hexane:ethyl acetate (8:1) and (3:1) to give the title compound (630mg).
δ (¹H, CDCl₃): 9.72 (s, 1H, CHO), 6.98 (s, 1H, CHPh₂), 6.06 (dd, 1H, H-2, J=1.2 and 3.3 Hz), 4.84 (dd, 1H, H-2', J=3 and 4.2 Hz), 4.64 (d, 1H, H-1', J=1.5 Hz), 4.05 (m, 2H, H-3' and H-8a-CH₂), 3.73 (m, 2H, H-5' and H-8a-CH₂), 3.29 (dd, 1H, H-4', J=2.7 and 9 Hz), 3.01 (t, 2H, OCH₂CH₂CH_{3,}, J= 7 Hz), 2.68 (t, 1H, H-1, J=3.9 Hz), 2.44 (s, 3H, p-Ts).

### INTERMEDIATE 18

### [1R-(1α, 3aβ, 4β, 4aβ, 7β, 7aα, 8aβ)] 8a-[(2,3-Anhydro-6-deoxy-4-O-methyl-β-D-mannopyranosyloxy) methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A suspension of sodium hydride (3.2 mmol) in dry dimethylformamide (6ml), under nitrogen atmosphere, was treated with a solution of Intermediate 16 (1.6mmol) in dry dimethylformamide (6ml). After 40 minutes, water:ethyl acetate (1:1 ; 60ml) was added. The organic phase was evaporated and the residue purified by flash chromatography using hexane:ethyl acetate (4:1) as eluent to give the title compound (960mg), having the identical proton NMR spectrum as the compound of Intermediate 3.

### INTERMEDIATE 19

### 1R-(1α, 3aβ, 4β, 4aβ, 7β, 7aα, 8aβ)] 8a-[(2,3-Anhydro-6-deoxy-4-O-propyl-β-D-mannopyranosyloxy) methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a suspension of sodium hydride (35mg) in dry dimethylformamide (10ml) was added dropwise a solution of intermediate 17 (600mg) in dry dimethylformamide (15ml). The mixture was stirred at room temperature under nitrogen for 1h and was then treated with 1N ammonium chloride (50ml) and ethyl acetate (70ml). The organic layer was washed with water and brine, dried over anhydrous magnesium sulphate and evaporated to dryness. The residue was chromatographed on a silca gel flash column eluting with hexane:ethyl acetate (8:1) and (6:1) to yield the title compound (450mg) as a white foam.
δ (¹H, CDCl₃): 9.75 (s, 1H, CHO), 6.99 (s, 1H, CHPH₂), 6.08 (dd, 1H- H-2, J=1.5 and 4.8 Hz), 4.68 (s, 1H, H-1'), 4.11 and 3.79 (2d, 2H, 8a-CH₂, J=9.0 Hz), 3.68 (m, 1H, H-5'), 3.45 (m, 1H, H-4'), 3.24 and 3.12 (2d, 2H, H-2', H-3', J=3.9 Hz), 3.19 (t, 2H, OCH₂CH₂CH₃, J=7 Hz), 2.86 (t, 1H, H-1, J=3.9 Hz).

### INTERMEDIATE 20

### [1R-(1α, 3aβ, 4β, 4aβ, 7β, 7aα, 8aβ)] 8a-[(6-Deoxy-3-O-tosyl-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a suspension of 10% palladium on charcoal (800mg) in ethyl acetate (60ml) in a hydrogenation bottle under nitrogen were added a solution of Intermediate 14 (1.0g) in ethyl acetate (30ml) and a mixture of methanol:1N hydrochloric acid (3:1; 4ml). The bottle was shaken in a Parr hydrogenation apparatus at a pressure of 30 psi of hydrogen for 18 hours. The reaction mixture was filtered and the filtrate was evaporated to dryness. The residue was purified by flash column chromatography on silica gel, eluting with dichloromethane : methanol (20:1). The appropriate fractions were combined and the solvent was evaporated to give the title compound (380mg).
δ (¹H, CDCl₃): 9.70 (s, 1H, CHO), 7.84 and 7.38 (2d, 4H, Ts), 6.06 (dd, 1H, H-2, J=1.5 and 3.6 Hz), 4.75 (dd, 1H, H-3', J=3.3 and 4.5 Hz), 4.61 (d, 1H, H-1', J=1.2 Hz), 4.05 (d, 1H, H-8aCH₂a, J=9.3 Hz), 3.92 (dd, 1H, H-2', J=1.2 and 4.5 Hz), 3.65 (m, 3H, H-5', H-4' and H-8aCH₂b), 2.63 (m, 1H, H-1), 2.46 (s, 3H, Ts).

### INTERMEDIATE 21

### [1R-(1α, 3aβ, 4β, 4aβ, 7β, 7aα, 8aβ)] 8a-[(2,3-Anhydro-6-deoxy-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A solution of Example 7 (585mg) in dry dichloromethane (20ml) was treated dropwise with a purple solution of diphenyldiazomethane in dichloromethane (0.35 M, 8ml). The resulting solution was stirred at room temperature for 14 hours. The solvent was removed under reduce pressure and the residue was purified by flash column chromatography on silica gel eluting with hexane:ethyl acetate (6:1) and (4:1). The appropriate fractions were combined and evaporated to yield the title compound (764mg) as a white foam.
δ (¹H, CDCl₃): 9.75 (s, 1H, CHO), 7.26-7.44 (m, 10H, 2Ph), 7.00 (s, 1H, CO₂CHPh₂), 6.09 (dd, 1H, H-2, J=1.2 and 3.6 Hz), 4.71 (s, 1H, H-1'), 3.79 and 4.12 (2d, 2H, 8aCH₂, J=9.3 Hz), 3.63 (dd, 1H, H-4', J=5.7 and 8.7 Hz), 3.21 (m, 1H, H-5'), 3.15 and 3.24 (2d, 2H, H-2' and H-3', J=3.6 Hz), 2.86 (t, 1H, H-1, J=3.9 Hz).

### INTERMEDIATE 22

### [1R-(1α, 3aβ, 4β, 4aβ, 7β, 7aα, 8aβ)] 8a-[(2,3-Anhydro-4,6-dideoxy-4-fluoro-β-D-talopyranosyloxy) methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A solution of Intermediate 21 (300mg) in dichloromethane (25ml) was treated with diethylaminosulfur trifluoride (0.16ml), and the resulting mixture was stirred overnight at room temperature. The mixture was cooled to 0^{o}C, quenched by addition of methanol (15ml) and then concentrated under reduced pressure. Flash chromatography of the residue on silica gel eluting with hexane:ethyl acetate (6:1) and (4:1) afforded the title compound (160mg).
δ (¹H, CDCl₃): 9.73 (s, 1H, CHO), 7.45 - 7.26 (m, 10H, 2Ph), 6.98 (s, 1H, CHPh₂),6.09 (dd, 1H, H-2, J= 1.5 and 3.9 Hz), 5.09 (d, 1H, H-1', J= 1.5 Hz), 4.75 and 4.58 (2dq, 1H, H-5', J_{5',F}=48.6 Hz, J= 6.6 Hz, J= 3.9 Hz), 4.18 and 4.11 (2d, 1H, H-4', J_{4'F}=23.1 Hz, J=3.9 Hz), 3.97 and 3.81 (2d, 2H, 8aCH₂, J=9.3 Hz), 3.75 and 3.70 (2m, 2H, H-2' and H-3'), 2.84 (t, 1H, H-1).

### INTERMEDIATE 23

### [1R-(1α, 3aβ, 4β, 4aβ, 7β, 7aα, 8aβ)] 8a-[(2,3-Anhydro-6-deoxy-4-O-(2-methoxyethyl)-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a suspension of sodium hydride (24mg) in dry tetrahydrofuran (20ml) at 0^{o}C under nitrogen was added Intermediate 21 (300mg) and the mixture was stirred for 30 minutes. 2-Bromoethyl methyl ether (300mg) and 1N tetrabutylammonium iodide (2ml) were added and the mixture was heated at 40^{o}C for 2 days. The reaction was quenched with 1N ammonium chloride (10ml) and the mixture diluted with ethyl acetate (30ml). The organic layer was washed with water and brine, dried over anhydrous magnesium sulphate and the solvent evaporated to dryness. The residue was chromatographed on a silica gel flash column eluting with hexane:ethyl acetate (5:1) to give the title compound (? mg) as a colourless oil.
δ (¹H, CDCl₃): 9.75 (s, 1H, CHO), 6.98 (s, 1H, CHPh₂), 6.08 (dd, 1H, H-2, J=1.2 and 3.3 Hz), 4.67 (s, 1H, H-1'), 4.10 and 3.78 (2d, 2H, 8a-CH₂, J=9.0 Hz) 4.09-4.00 (m, 2H, H-4', H-5'), 3.90-3.53 (m, 4H, OCH₂CH₂OCH₃), 3.40 (s, 3H, CH₃OCH₂CH₂), 3.29 and 3.11 (2d, 2H, H-3', and H-2', J=3.9 and 3.9 Hz), 2.86 (t, 1H, H-1, J=3.7 Hz).

### INTERMEDIATE 24

### [1R-(1α, 3aβ, 4β, 4aβ, 7β, 7aα, 8aβ)] 8a-[(2,3-Anhydro-6-deoxy-4-O-(2-methylprop-2-enyl)-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A mixture of Intermediate 21 (0.3 mmol), cesium carbonate (0.3 mmol) and 3-bromo-2-methyl-propene (0.5 mmol) in dry dimethylformamide (1.5ml) was stirred for three days at room temperature. After diluting with diethyl ether (30ml) the mixture was washed with water. The solvent was evaporated and the residue was purified by flash chromatography using hexane:ethyl acetate (4:1) as eluent to give the title compound (62mg).
δ (¹H, CDCl₃): 9.75 (s, 1H, CHO), 7.35 (m, 10H, 2xPh), 6.99 (s, 1H, CHPh₂), 6.08 (dd, 1H, H-2, J=1.5 and 3.6 Hz), 5.00 and 4.93 (m,m, 1H, 1H, CH₂=C), 4.70 (s, 1H, H-1'), 4.12 and 3.79 (2d, 2H, 8a-CH₂, J=9.3 Hz), 4.12 and 3.96 (d,d, 1H, 1H, CH₂O, J=12 Hz), 3.26 (m, 3H, H-3', H-4' and H-5'), 3.13 (d, 1H, H-2', J=3.9 Hz), 2.86 (t, 1H, H-1, J=3.9 Hz), 2.23 (m, 1H, CH(CH₃)₂).

### INTERMEDIATE 25

### [1R-(1α, 3aβ, 4β, 4aβ, 7β, 7aα, 8aβ)] 8a-[(2,3-Anhydro-6-deoxy-4-O-(1-methylethyl)carbonyl-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a solution of Intermediate 21 (160mg) in dry dichloromethane (5ml) were added 4-dimethylaminopyridine (70mg) and isobutyryl chloride (60µl). The mixture was stirred overnight at room temperature and then concentrated to give a yellow oil, which was chromatographed on a silica gel flash column using hexane:ethyl acetate (5:1) as eluent to provide the title compound (162mg) as a white foam.
δ (¹H, CDCl₃): 9.75 (s, 1H, CHO), 7.26-7.44 (m, 10H, 2Ph), 6.99 (s, 1H, CHPh₂), 6.08 (dd, 1H, H-2), 5.75 (s, 1H, H-1'), 4.68 (d, 1H, H-4', J=9 Hz), 4.12 and 3.80 (2d, 2H, 8aCH₂, J=9 Hz), 3.43 (dq, 1H, H-5', J=6.6 and 9 Hz), 3.15 (s, 2H, H-2' and H-3'), 2.86 (m, 1H, H-11), 2.59 (m, 1H, 4'-OCOCHMe₂).

### INTERMEDIATE 26

### [1R-(1α, 3aβ, 4β, 4aβ, 7β, 7aα, 8aβ)] 8a-[(2,3-Anhydro-6-deoxy-4-O-(2,2-dimethylpropionyl)-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A mixture of Intermediate 21 (0.3 mmol) and 4-dimethylaminopyridine (0.6 mmol) in dry dichloromethane (15ml) was treated with pivaloyl chloride (0.45 mmol). After 2.5 hours, the mixture was washed with water. The organic phase was evaporated and the residue was purified by flash column chromatography using hexane:ethyl acetate (5:1) as eluent to give the title compound (190mg).
δ (¹H, CDCl₃): 9.76 (s, 1H, CHO), 7.83 (m, 10H, 2xPh), 7.00 (s 1H, CHPh₂), 6.08 (dd, 1H, H-2, J=1.5 and 3.3 Hz), 4.77 (s, 1H, H-1'), 4.67 (d, 1H, H-4', J= 9 Hz), 4.13 and 3.81 (d,d, 1H, 1H, 8a-CH₂, J=9 Hz), 3.43 (m, 1H, H-5'), 3.13 (m, 2H, H-2' and H-3'), 2.86 (t, 1H, H-1, J= 3.9 Hz), 2.24 (m, 1H, CH(CH₃)₂).

### INTERMEDIATE 27

### [1R-(1α, 3aβ, 4β, 4aβ, 7β, 7aα, 8aβ)] 8a-[(2,3-Anhydro-4-O-benzyloxy-carbonyl-6-deoxy-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A solution of Intermediate 21 (0.3 mmol) and 4-dimethylaminopyridine (0.8mmol) in dry dichloromethane (15ml) was treated dropwise with benzyloxycarbonyl chloride (0.7 mmol) and stirred for 3 hours. After washing with water and brine, the solvent was evaporated and the residue was purified by flash chromatography using hexane:ethyl acetate (5:1) as eluent to give the title compound (185mg).
δ (¹H, CDCl₃): 9.75 (s, 1H, CHO), 7.32 (m, 15H, 3xPh), 6.99 (s, 1H, CHPh₂), 6.08 (dd, 1H, H-2, J=1.2 and 3.3 Hz), 5.20 (m, 2H, OCH₂Ph), 4.72 (s, 1H, H-1'), 4.57 (d, 1H, H-4', J=8.4 Hz), 4.11 and 3.79 (2d, 2H, 8a-CH_{2,}, J=9 Hz), 3.43 (m, 1H, H-5'), 3.27 and 3.15 (2d, 2H, H-2' and H-3', J=3.6 Hz), 2.85 (t, 1H, H-1, J=3.9 Hz), 2.24 (m, 1H, CH(CH₃)₂).

### INTERMEDIATE 28

### [1R-(1α, 3aβ, 4β, 4aβ, 7β, 7aα, 8aβ)] 8a-[(2,3-Anhydro-6-deoxy-4-oxo-β-D-mannopyranosyloxy) methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A solution of trifluoroacetic anhydride (0.1ml) in dry dichloromethane (5ml) at -60^{o}C was treated dropwise with dimethylsulfoxide (0.06ml) and a solution of Intermediate 21 (0.39 mmol) in dry dichloromethane (5ml). After 60 minutes, triethylamine (0.24ml) was added and the mixture was stirred at -20^{o}C for 2 hours. The mixture was diluted with dichloromethane (20ml) and washed with water. After removal of the solvent, the residue was purified by flash chromatography using hexane:ethyl acetate (4:1) as eluent to give the title compound (171mg).
δ (¹H, CDCl₃): 9.76 (s, 1H, CHO), 7.36 (m, 10H, 2xPh), 6.59 (s, 1H, CHPh₂), 6.09 (dd, 1H, H-2, J=1.5 and 3.6 Hz), 4.84 (s, 1H, H-1'), 4.11 and 3.84 (2d, 2H, 8a-CH_{2,}, J=9 Hz), 3.92 (q, 1H, H-5', J=6.9 Hz), 3.59 and 3.37 (2d, H-2' and H-3', J=4.2 Hz), 2.86 (t, 1H, H-1, J=3.9 Hz), 2.26 (m, 1H, CH(CH₃)₂).

### EXAMPLE 1

### [1R-(1α, 3aβ, 4β, 4aβ, 7β, 7aα, 8aβ)] 8a-[(2,3-Anhydro-6-deoxy-4-O-methyl-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 3 (140mg) in ethyl acetate (20ml) was added 10% palladium on charcoal (100mg) under nitrogen. The mixture was shaken in a Parr apparatus under 15 psi hydrogen for 1 hour at room temperature. The catalyst was filtered off and the solvent evaporated to dryness. The residue was purified by flash chromatography on silica gel, eluting with hexane:ethyl acetate (5:1) and dichloromethane:methanol (20:1). The appropiate fractions were combined and the solvent removed to give the title compound (85mg) as a foam.
δ (¹H, CDCl₃): 9.76 (s, 1H, CHO), 6.08 (dd, 1H, H-2, J=1.2 and 3.3 Hz), 4.74 (s, 1H, H-1'), 3.63 and 4.13 (2d, 2H, 8aCH₂, J=9.3 Hz), 3.48 (m, 4H, H-4' and 4'-OMe), 2.26 (d, 1H, H-2', J=3.9 Hz), 3.19 (m, 1H, H-5'), 3.13 (d, 1H, H-3', J=3.9 Hz), 3.06 (d, 1H, H-4', J= 8.7 Hz), 2.74 (t, 1H, H-1, J=3.6 Hz); δ (¹³C, CDCl₃): 204.8 (CHO), 175.6 (CO₂H), 148.2 (C-3), 130.8 (C-2), 98.0 (C-1'), 76.5 (C-4'), 74.3 (8aCH₂), 74.4 (C-3a), 72.5 (C-5'), 65.6 (C-8a), 59.0 (C-4), 58.2 (4'-OMe), 53.6 (C-2'), 50.3 (C-3'), 46.4 (C-1).

### EXAMPLE 2

### [1R-(1α, 3aβ, 4β, 4aβ, 7β, 7aα, 8aβ)] 8a-[(2,3-Anhydro-6-deoxy-4-O-methyl-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, sodium salt

Example 1 (310mg) was dissolved in methanol (10ml) and an aqueous sodium hydroxide solution (0.0976N, 6.69ml) was added. The mixture was stirred for 2 hours at room temperature. The solvent was removed and the residue was dissolved in water (2ml) and freeze-dried to yield the title compound (324mg) of as white solid.
δ (¹H, CDCl₃): 9.87 (s, 1H, CHO), 5.98 (dd, 1H, H-2, J=1.5 and 3.6 Hz), 4.69 (s, 1H, H-1'), 3.85 and 4.07 (2d, 2H, 8aCH₂, J=9.6 Hz), 3.47 (s, 3H, 4'-OMe), 3.26 (d, 1H, H-2', J=3.9 Hz), 3.19 (dq, 1H, H-5', J=5.7 and 9 Hz), 3.12 (d, 1H, H-3', J=3.9 Hz), 2.97 (dd, 1H, H-4', J=0.6 and 8.7 Hz), 2.66 (t, 1H, H-1, J=3.6 Hz); δ (¹³C, CDCl₃): 209.6 (CHO), 178.6 (CO₂H), 152.4 (C-3), 130.2 (C-2), 100.1 (C-1'), 78.2 (C-4'), 77.9 (8aCH₂), 73.8 (C-3a), 73.9 (C-5'), 65.6 (C-8a), 59.2 (C-4), 58.2 (4'-OMe), 54.6 (C-2'), 51.9 (C-3'), 46.4 (C-1).

### EXAMPLE 3

### [1R-(1α, 3aβ, 4β, 4aβ, 7β, 7aα, 8aβ)] 8a-[(2,3-Epithio-4-O-methyl-2,3,6-trideoxy-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

A mixture of Intermediate 5 (0.67mmol), 5,5-dimethyl-2-thiolo-2-thioxo-1,3,2-dioxaphosphorinane¹ (3.37mmol) and triethylamine (4.02 mmol) in dry dimethylformamide (5ml) was heated for 48 hours at 100^{o}C . After cooling, the mixture was poured into aqueous ether. The organic phase was evaporated and the residue purified by flash chromatography using dichloromethane:methanol (30:1) as eluent to give the title compound (180mg).
δ (¹H, CDCl₃): 9.77 (s, 1H, CHO), 6.08 (dd, 1H, H-2, J=1,2 and 3.3 Hz), 4.95 (d, 1H, H-1', J=1.8 Hz), 4.15 and 3.65 (d,d, 1H, 1H, 8a-CH₂), J= 9.3 Hz), 3.33 (d, 1H, H-4', J= 8.7 Hz), 3.18 (m, 1H, H-5'),3.14 (m, 2H, H-2' and H-3'), 2.70 (m, 1H, H-1),2.30 (m, 1H, CH(CH₃)₂); δ (¹³C, CDCl₃): 204.7 (CHO), 175.6 (CO₂H),148.3 (C-3),130.7 (C-2),98.5 (C-1'), 79.1 (C-4'), 74.1 (8a-CH₂), 73.5 (C-5'), 46.6 (C-1),35.6 (C-2'),35.2 (C-3'),27.5 (CH(CH₃)₂).

¹ R.S. Edmundson; Tetrahedron, 21, 2379 (1965).

### EXAMPLE 4

### [1R-(1α, 3aβ, 4β, 4aβ, 7β, 7aα, 8aβ)] 8a-[(2,3-Anhydro-6-deoxy-4-O-propyl-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 19 (400mg) in ethyl acetate (100ml) was added 10% palladium on charcoal (200mg) under nitrogen. The mixture was shaken in a Parr apparatus under 20 psi of hydrogen for 1 hour at room temperature. The catalyst was filtered off and the solvent evaporated to dryness. The residue was purified on a silica gel flash column eluting with methylene chloride and methylene chloride: methanol (25:1) to obtain the title compound (300mg) as a white foam.
δ (¹H, CDCl₃): 9.76 (s, 1H, CHO), 6.08 (dd, 1H, H-2, J=1.5 and 3.6 Hz), 4.72 (s, 1H, H-1'), 4.13 and 3.65 (2d, 2H, 8a-CH₂, J=9 Hz), 3.67 (m, 1H, H-5'), 3.44 (m, 1H, H-4'), 3.25 and 3.12 (2d, 2H, H-2', H-3', J=4.2 Hz), 3.20 (m, 2H, OCH₂CH₂CH₃), 2.74 (t, 1H, H-1, J=3.9 Hz); δ (¹³C, CDCl₃): 204.8 (CHO), 175.8 (CO₂H), 130.8 (C-2), 148.2 (C-3), 98.0 (C-1'), 74.9 (C-4'), 74.3 (C-8a-CH₂), 72.6 (C-5'), 72.4 (OCH₂CH₂CH₃ en C-4'), 65.6 (C-8a), 59.0 (C-3), 54.2 (C-2'), 50.3 (C-3'), 18.6 (C-6').

### EXAMPLE 5

### [1R-(1α, 3aβ, 4β, 4aβ, 7β, 7aα, 8aβ)] 8a-[(2,3-Anhydro-6-deoxy-4-O-propyl-β-D-mannopyranosyloxy) methyl]-4-formyl-4,4a,5,6,7,7a,8,8a-octahydro-7-methyl-3-(1-methylethyl)-1,4-methano-s-indacene-3a(1H)-carboxylic acid, sodium salt

To a solution of Example 4 (400mg) in dry methanol (30ml) was added dropwise 0.0976N sodium hydroxide solution (8.15ml) at room temperature. The mixture was stirred for 1 hour. The solvent was evaporated to dryness and the solid residue dissolved in the minimum volume of water (10-15ml). This solution was lyophilized to obtain the title compound (417mg) as a white solid.
δ (¹H, CDCl₃): 9.89 (s, 1H, CHO), 5.97 (dd, 1H, H-2, J=1.5 and 3.6 Hz), 4.70 (s, 1H, H-1'), 4.09 and 3.86 (2d, 2H, 8a-CH₂, J=9.7Hz), 3.70 (m, 1H, H-5'), 3.44 (m, 1H, H-4'), 3.24 and 3.12 (2d, 2H, H-2' and H-3', J= 4 Hz), 3.20 (m, 2H, OCH₂CH₂CH₃), 2.65 (t, 1H, H-1, J=3.9 Hz); δ (¹³C, CDCl₃): 209.6 (CHO), 178.38 (CO₂Na), 152.4 (C-3), 130.1 (C-2), 100.1 (C-1'), 77.9 (OCH₂CH₂CH₃ en C-4'), 76.6 (C-4'), 73.5 (C-5'), 73.1 (C-8a-CH₂), 66.6 (C-3'), 18.9 (C-6').

### EXAMPLE 6

### [1R-(1α, 3aβ, 4β, 4aβ, 7β, 7aα, 8aβ)] 8a-[(2,3-Anhydro-4-O-benzyl-6-deoxy-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a suspension of sodium hydride (22mg) in dry dimethylformamide (5ml) was added dropwise a solution of Intermediate 15 (200mg) in dry dimethylformamide (7ml) and the mixture was stirred for 1 hour at room temperature. The reaction mixture was neutralized with 1N hydrochloric acid and ethyl acetate (30ml) was added. The organic layer was washed with water and brine, dried over anhydrous magnesium sulphate, filtered and evaporated to dryness. The residue was chromatographed on a silica gel flash column using methylene chloride:methanol (50:1) and (35:1) as eluents to give the title compound as a white foam (150mg).
δ (¹H, CDCl₃): 9.86 (s, 1H, CHO), 7.31 (m, 5H PhCH₂O), 6.08 (dd, 1H, H-2, J=1.2 and 3.3 Hz), 4.75 (d, 1H, OCH₂Ph, J= 11.4 Hz), 4.73 (s, 1H, H-1'), 4.56 (d, 1H, OCH₂Ph, J= 11.4 Hz), 4.17 and 3.62 (2d, 2H, 8a-CH₂, J=9.0 Hz), 3.32 and 3.14 (2d, 2H, H-3' and H-2', J=3.7 Hz), 3.30 (m, 2H, H-4', H-5'), 2.71 (t, 1H, H-1); δ (¹³C, CDCl₃): 204.9 (CHO), 175.8 (COOH), 137.2, 128.14, 128.11 (6C-Ph), 148.3 (C-3), 130.8 (C-2), 98.0 (C-1'), 74.4 (C8a-CH₂), 74.3 (C-4'), 72.6 (OCH₂Ph), 72.5 (C-5'), 65.5 (C-8a), 54.0 (C-2'), 50.3 (C-3'), 18.6 (C-6').

### EXAMPLE 7

### [1R-(1α, 3aβ, 4β, 4aβ, 7β, 7aα, 8aβ)] 8a-[(2,3-Anhydro-6-deoxy-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a suspension of dry sodium hydride (75mg) in dry dimethylformamide (5ml) under nitrogen was added a solution of Intermediate 20 (500mg) in dry dimethylformamide (5ml). The reaction mixture was stirred under nitrogen for 3 hours and the reaction was then quenched by water addition and neutralized to pH 6.5-7 with 0.5 M aqueous hydrochloric acid. The mixture was concentrated and the residue partitionated in water:ethyl acetate (1:1;100ml). The aqueous phase was extracted with ethyl acetate (2x20ml), and the organic phases were combined and treated with brine and dried over magnesium sulphate, filtered and concentrated in vacuo to a syrup. This was purified by flash column chromatography on silica gel eluting with dichloromethane: methanol (40:1) to give the title compound (172mg) as a white foam.
δ (¹H, CDCl₃): 9.76 (s, 1H, CHO), 6.09 (dd, 1H, H-12, J=1.5 and 3.6 Hz), 4.77 (s, 1H, H-1'), 3.61 and 4.21 (2d, 2H, 8aCH₂, J=9 Hz), 3.60 (d, 1H, H-4', J=8.7 Hz), 3.21 (dq, 1H, H-5', J=9 and 6 Hz), 3.16 and 3.27 (2d, 2H, H-2' and H-3' J=3.6 Hz), 2.70 (t, 1H, H-1); δ (¹³C, CDCl₃): 205.5 (CHO), 174.6 (CO₂H), 148.4 (C-3), 130.8 (C-2), 97.8 (C-1'), 74.1 (8aCH₂), 73.9 (C-4'), 73.7 (C-3a), 67.2 (C-5'), 65.5 (C-8a), 59.0 (C-4), 56.5 (C-2'), 50.4 (C-3'), 46.5 (C-1).

### EXAMPLE 8

### [1R-(1α, 3aβ, 4β, 4aβ, 7β, 7aα, 8aβ)] 8a-[(2,3-Anhydro-4,6-dideoxy-4-fluoro-β-D-talopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 22 (130mg) in ethyl acetate (30ml) was added 10% palladium on charcoal (100mg) under nitrogen. The mixture was shaken in a Parr apparatus under 15 psi of hydrogen for 1 hour at room temperature. The catalyst was filtered off and the solvent evaporated to dryness. The residue was chromatographed on a silica gel flash column using hexane:ethyl acetate (4:1) and dichloromethane:methanol (20:1) as eluents to give the title compound as a white foam (82mg).
δ (¹H, CDCl₃): 9.79 (s, 1H, CHO), 6.08 (dd, 1H, H-2, J= 1.2 and 3.6 Hz), 5.13 (s, 1H, H-1'), 4.75 and 4.58 (2dq, 1H, H-5', J_{5'F}=48.3 Hz, J=6.6 Hz, J=3.9 Hz), 4.18 and 4.11 (2d, 1H, H-4', J=3.9 Hz and J_{4'F}=23.1 Hz ), 4.07 and 3.61 (2d, 2H, 8aCH₂, J=9.3 HZ), 3.77 (m, 2H, H-2' and H-3'), 2.67 (t, 1H, H-1); δ (¹³C, CDCl₃): 204.6 (CHO), 175.3 (CO₂H), 148.2 (C-3), 130.7 (C-2), 101.7 (C-1'), 90.2 (d, C-4', J_{C4'-F}= 157 Hz), 80.3 (d, C-5', J_{C5'-F}= 21.4 Hz), 74.3 (C-8aCH₂), 73.4 (C-3a), 65.4 (C-8a), 58.9 (C-4), 55.9 (C-2'), 55.1 (d, C-3', J_{C3'-F}=6.3 Hz), 16.4 (d, C-6', J_{C6'-F}= 21 Hz).

### EXAMPLE 9

### [1R-(1α, 3aβ, 4β, 4aβ, 7β, 7aα, 8aβ)] 8a-[(2,3-Anhydro-6-deoxy-4-O-(2-methoxyethyl)-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 23 (140mg) in ethyl acetate (50ml) was added 10% palladium on charcoal (70mg) under nitrogen. The mixture was shaken in a Parr apparatus under 20 psi of hydrogen for 1 hour at room temperature. The catalyst was filtered off and the solvent evaporated to dryness. The residue was purified on a silica gel flash column eluting with dichloromethane and dichloromethane:methanol (40:1) to give the title compound (74mg) as a white foam.
δ (¹H, CDCl₃): 9.78 (s, 1H, CHO), 6.08 (dd, 1H, H-2, J=1.2 and 3.3 Hz), 4.74 (s, 1H, H-1'), 4.23 and 3.60 (2d, 2H, 8a-CH₂, J=9 Hz), 3.86-3.50 (m, 4H, OCH₂CH₂OCH₃), 3.38 (s, 3H, CH₃OCH₂CH₂O), 3.30 and 3.13 (2d, 2H, H-2' and H-3', J=3.9 Hz), 3.26-3.22 (m, 2H, H-4' and H-5'), 2.68 (t, 1H, H-1, J=3.7 Hz); δ (¹³C, CDCl₃): 204.8 (CHO), 175.2 (COOH), 148.3 (C-3), 130.8 (C-2), 97.9 (C-1'), 74.2 (C-8aCH₂), 72.4 (C-5'), 71.7 and 69.9 (OCH₂CH₂OCH₃), 54.1 (C-2'), 50.3 (C-3'), 46.5 (CH₃OCH₂CH₂), 18.5 (C-6').

### EXAMPLE 10

### [1R-(1α, 3aβ, 4β, 4aβ, 7β, 7aα, 8aβ)] 8a-[(2,3-Anhydro-6-deoxy-4-O-(2-methylpropyl)-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 24 (0.14 mmol) in ethyl acetate (30ml) was added 10% palladium on charcoal (50mg) under nitrogen. The mixture was shaken in a Parr apparatus under 25 psi of hydrogen for 30 minutes at room temperature. The catalyst was filtered off and the solvent evaporated to dryness. The residue was purified by flash chromatography using dichloromethane:methanol (40:1) as eluent to give the title compound (49mg).
δ (¹H, CDCl₃): 9.76 (s, 1H, CHO), 6.09 (dd, 1H, H-2, J=1.2 and 3.3 Hz), 4.73 (s, 1H, H-1'), 4.12 and 3.65 (2d, 2H, 8a-CH₂, J=9.3 Hz), 3.49 (m, 1H, H-5'), 3.22 (m, 3H, H-2' and CH₂-O), 3.13 (m, 2H, H-3' and H-4'), 2.75 (t, 1H, H-1, J=3.9 Hz), 2.33 (m, 1H, CH(CH₃)₂); δ (¹³C, CDCl₃): 204.8 (CHO), 176.0 (CO₂H), 148.2 (C-3), 130.8 (C-2), 98.0 (C-1'), 77.6 (C-1''), 75.2 (C-4'), 74.3 (8a-CH₂), 72.9 (C-3a), 72.6 (C-5'), 65.6 (C-8a), 59.0 (C-4), 54.1 (C-2'), 50.4 (C-3'), 46.4 (C-1), 41.7 (C-4a), 41.3 (C-7a).

### EXAMPLE 11

### [1R-(1α, 3aβ, 4β, 4aβ, 7β, 7aα, 8aβ)] 8a-[(2,3-Anhydro-6-deoxy-4-O-(1-methylethyl)carbonyl-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 25 (140mg) in ethyl acetate (20ml) was added 10% palladium on charcoal (120mg) under nitrogen. The mixture was shaken in a Parr apparatus under 20 psi of hydrogen for 1 hour at room temperature. The catalyst was filtered off and the solvent evaporated to dryness. The residue was purified by flash column chromatography on silica gel eluting with hexane:ethyl acetate (4:1) and dichlorometane:methanol (20:1) to afford the title compound (103mg) as a white foam.
δ (¹H, CDCl₃): 9.74 (s, 1H, CHO), 6.09 (dd, 1H, H-2, J= 1.5 and 3.6 Hz), 4.78 (s, 1H, H-1'), 4.66 (d, 1H, H-4', J=9 Hz), 4.10 and 3.60 (2d, 2H, 8aCH₂, J=9 Hz), 3.42 (dq, 1H, H-5', J=9 and 3 Hz), 3.15 (s, 2H, H-2' and H-3'), 2.78 (m, 1H, H-1), 2.58 (m, 1H, 4'-OCOCHMe₂); δ (¹³C, CDCl₃): 204.9 (CHO), 176.1, 175.9 (CO₂H and 4'OCOR), 148.2 (C-3), 130.9 (C-2), 97.9 (C-1'), 74.5 (C-8aCH₂), 72.6 (C-3a), 71.4 (C-4'), 67.8 (C-5'), 66.0 (C-8a), 59.0 (C-4), 18.5 and 18.8 (2CH₃ of 4'-OCOCHMe₂).

### EXAMPLE 12

### [1R-(1α, 3aβ, 4β, 4aβ, 7β, 7aα, 8aβ)] 8a-[(2,3-Anhydro-6-deoxy-4-O-(2,2-dimethylpropionyl)-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 26 (0.21 mmol) in ethyl acetate (60ml) was added 10% palladium on charcoal (50mg) under nitrogen. The mixture was shaken in a Parr apparatus under 20 psi of hydrogen for 30 minutes at room temperature. The catalyst was filtered off and the solvent evaporated to dryness. The residue was purified by flash chromatography using dichloromethane:methanol (40:1) as eluent to give the title compound (101mg).
δ (¹H, CDCl₃): 9.75 (s, 1H, CHO), 6.10 (dd, 1H, H-2, J= 1.2 and 3.3 Hz), 4.80 (s, 1H, H-1'), 4.64 (d, 1H, H-4', J= 9 Hz), 4.11 and 3.70 (2d, 2H, 8a-CH₂, J= 9.3 Hz), 3.43 (m, 1H, H-5'), 3.13 (m, 2H, H-2' and H-3'), 2.78 (t, 1H, H-1, J= 3.9 Hz), 2.34 (m, 1H, CH(CH₃)₂); δ (¹³C, CDCl₃): 204.9 (CHO), 177.3 and 176.5 (2xCO₂), 148.2 (C-3), 130.9 (C-2), 98.0 (C-1'), 74.5 (8a-CH₂), 72.6 (C-2), 71.3 (C-5'), 67.9 (C-4'), 65.7 (C-8a), 59.0 (C-4), 54.3 (C-2'), 50.1 (C-3'), 46.3 (C-1), 41.8 ( C-4a), 41.3 (C-8), 38.8 (C-^{t}Bu), 32.0 (C-5), 30.9 (C-7), 18.5 (^{t}Bu).

### EXAMPLE 13

### [1R-(1α, 3aβ, 4β, 4aβ, 7β, 7aα, 8aβ)] 8a-[(2,3-Anhydro-4-O-benzyloxycarbonyl-6-deoxy-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

A solution of Intermediate 27 (0.22 mmol) in dichloromethane (10ml) at 0^{o}C was treated with trifluoroacetic acid (0.1ml). After two hours, the mixture was washed with water and the organic phase was evaporated. The residue was purified by flash chromatography using dichloromethane: methanol (40:1) as eluent to give the title compound (60mg).
δ (¹H, CDCl₃): 9.74 (s, 1H, CHO), 7.37 (m, 5H, Ph), 6.09 (dd, 1H, H-2, J=1.5 and 3.6 Hz), 5.20 (m, 2H, CH₂Ph), 4.75 (s, 1H, H-1'), 4.55 (d, 1H, H-4', J=9 Hz), 4.08 and 3.70 (2d, 2H, 8a-CH₂, J=9 Hz), 3.47 (m, 1H, H-5'), 3.28 and 3.16 (d,d, 1H, 1H, H-2' and H-3', J=3.6 Hz), 2.79 (t, 1H, H-1, J=3.9 Hz), 2.33 (m, 1H, CH(CH₃)₂); δ (¹³C, CDCl₃): 204.8 (CHO), 176.4 (CO₂H), 154.1 (CO₃), 148.1 (C-3), 134.6 (Cipso), 130.9 (C-2), 128.8, 128.7, 128.5, 128.4 (Ph), 97.8 (C-1'), 74.5 (8a-CH₂), 72.5 (C-3a), 71.6 (C-5'), 71.1 8C-4'), 70.3 (CH₂Ph), 65.7 (C-8a), 58.9 (C-4), 53.9 (C-2'), 50.0 (C-3'), 46.2 (C-1), 41.7 (C-4a), 41.3 (C-7a).

### EXAMPLE 14

### [1R-(1α, 3aβ, 4β, 4aβ, 7β, 7aα, 8aβ)] 8a-[(2,3-Anhydro-6-deoxy-4-oxo-β-D-mannopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 28 (0.26 mmol) in ethyl acetate (30ml) was added 10% palladium on charcoal (50mg) under nitrogen. The mixture was shaken in a Parr apparatus under 25 psi of hydrogen for 1.5h at room temperature. The catalyst was filtered off and the solvent evaporated to dryness. The residue was purified by flash chromatography using dichloromethane:methanol (40:1) as eluent and appropriate fractions were collected and evaporated to give the title compound (38mg).
δ (¹H, CDCl₃): 9.72 (s, 1H, CHO), 6.12 (dd, 1H, H-2, J=1.5 and 3.6 Hz), 4.87 (s, 1H, H-1'), 4.10 and 3.76 (2d, 2H, 8aCH₂, J=9 Hz), 3.83 (q, 1H, H-5', J=6.6 Hz), 3.60 and 3.38 (d,d, 1H, 1H, 3' and 2', J=3.9 Hz), 2.82 (t, 1H, H-1, J=3.6 Hz), 2.36 (m, 1H, CH(CH₃)₂); δ (¹³C, CDCl₃): 204.9 (CHO), 202.9 (CO-4'), 176.2 (CO₂H), 148.2 (C-3), 130.9 (C-2), 96.1 (C-1'), 76.6 (C-5'), 74.7 (8aCH₂), 72.1 (C-3a), 65.7 (C-8a), 58.9 (C-4), 54.2 and 53.5 (C-2' and C-3'), 46.1 (C-1), 41.8 (C-4a), 41.3 (C-7a).

### EXAMPLE 15

### Characteristics of IMI 362184

IMI 362184 is a mutant of Sordaria araneosa (ATCC 36386, NRRL 3196) isolated following N-methyl-N'-nitro-N-nitrosoguanidine mutagenesis of ascospores of this strain. The characteristics of IMI 362184 are essentially similar to those described in British Patent Specification No. 1,162,027 for NRRL 3196, except that IMI 362184 produces 4'-demethylsordarin as a major product under the same conditions used for sordarin production by NRRL 3196.

## Claims

1. A compound of formula (I) 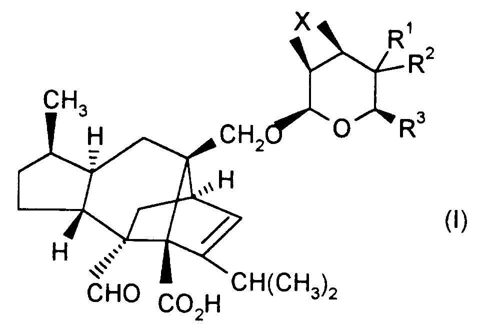 and pharmaceutically acceptable salts and solvates (e.g. hydrates) thereof, wherein R¹ represents hydrogen, halogen, C₁₋₆alkyl, hydroxy, C₁₋₆alkoxy, C₁₋₄alkoxyC₁₋₄alkoxy, arylC₁₋₄alkoxy or a group OCOR⁴ (where R⁴ is arylC₁₋₄alkoxy or a C₁₋₁₀alkyl group optionally containing one or two double bonds) and R² represents hydrogen or R¹ and R² may together with the carbon atom to which they are attached represent C=O;
R³ represents CH₂R⁵ (where R⁵ is hydrogen, hydroxyl, C₁₋₄alkoxy or a group OCOR⁶ in which R⁶ is C₁₋₄alkyl); and
X represents an oxygen or sulphur atom or a CH₂ group.

2. A compound as claimed in Claim 1 wherien R² is hydrogen and R¹ is sited in the α configuration

3. A compound as claimed in Claims 1 or 2 wherein R¹ represents C₁₋₄alkoxy, benzyloxy or OCOR⁴ (where R⁴ is a C₁₋₄alkyl group).

4. A compound as claimed in any of Claims 1 to 3 wherin R³ represents methyl.

5. A compound as claimed in any of Claims 1 to 4 wherein X represents oxygen.

6. A compound of formula (Ia) 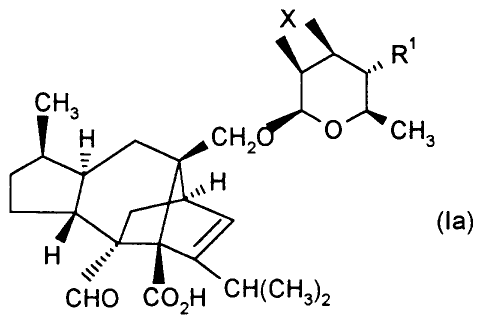 and pharmaceutically acceptable salts and solvates (e.g. hydrates) thereof, wherein X is oxygen and R¹ is a group selected from C₁₋₄alkoxy, benzyloxy or OCOR⁴ (where R⁴ is a C₁₋₄alkyl group).

7. The compounds;
[1R-(1α, 3aβ, 4β, 4aβ, 7β, 7aα, 8aβ)] 8a-[(2,3-anhydro-6-deoxy-4-O-propyl-β-D-mannopyranosyloxy)methyl]-4-formyl-4,4a,5,6,7,7a,8,8a-octahydro-7-methyl-3-(1-methylethyl)-1,4-methano-*s*-indacene-3a(1H)-carboxylic acid;
[1R-(1α, 3aβ, 4β, 4aβ, 7β, 7aα, 8aβ)] 8a-[(2,3-anhydro-6-deoxy-4-O-methyl-β-D-mannopyranosyloxymethyl]-4-formyl-4,4a,5,6,7,7a,8,8a-octahydro-7-methyl-3-(1-methylethyl)-1,4-methano-*s*-indacene-3a(1H)-carboxylic acid;
and pharmaceutically acceptable salts and solvates (e.g. hydrates) thereof.

8. A compound as claimed in any of Claims 1 to 7 for use in therapy.

9. The use of a compound as claimed in any of Claims 1 to 7 in the manufacture of a medicament for the treatment of fungal infections.

10. A pharmaceutical composition comprising a compound as claimed in any of Claims 1 to 7 in admixture with one or more physiologically acceptable carriers or excipients.

11. A method of treatment of the human or non-human animal body to combat fungal diseases which method comprises administering to said body an effective amount of a compound a claimed in any of Claims 1 to 7.

12. A process for the preparation of compounds of formula (I) which comprises
(a) cyclising a compound of formula (II) 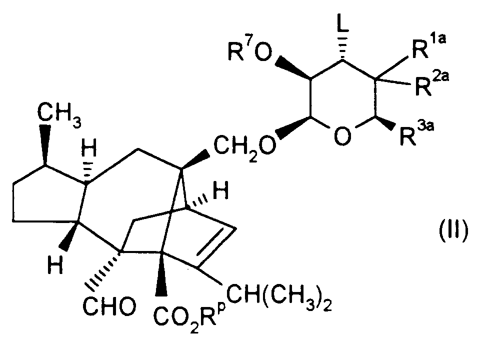 (in which R^{1a}, R^{2a} and R^{3a} are as defined for R¹, R² and R³ in formula (I) above or are protected derivatives thereof, R^{p} is hydrogen or a carboxyl protecting group, L is a suitable leaving group such as an alkyl- or arylsulphonyloxy group and R⁷ is hydrogen or OR⁷ is the same group as defined for L),
(b) The preparation of a compound of formula (I) by reacting a compound of formula (IV) 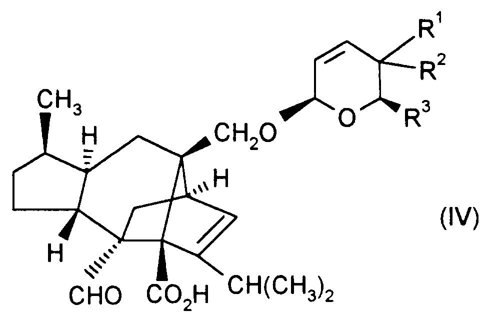 or a protected derivative thereof with a reagent capable of donating an oxygen atom or a CH₂ group, followed by the removal of any protecting groups present.
(c) The preparation of a compound of formula (I) in which X is sulphur by treating a compound of formula (III) 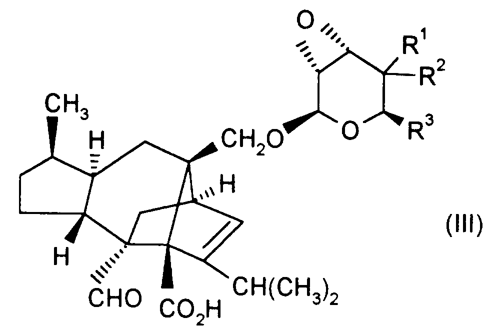 (in which R¹, R² and R³ are as defined in formula (I) above) with a sulphur donor;
followed where necessary or desired by one or more of the following steps:
(i) conversion of one compound of formula (I) or a protected derivative thereof into another compound of formula (I)
(ii) removal of one or more protecting groups.

13. A compound of formula (V) 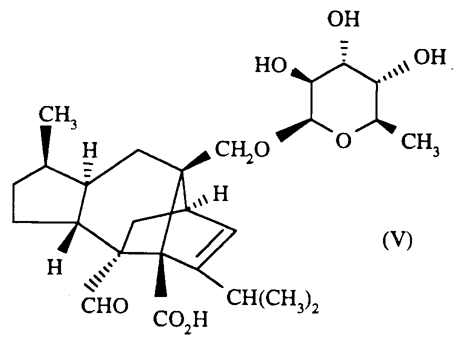 or a protected derivative thereof

14. A process for the preparation of a compound of formula (V) which process comprises:
(a) cultivating a microorganism capable of producing the compound of formula (V) and thereafter isolating the compound of formula (V) from the culture,
(b) the biotransformation of sordarin.
